# EUROPEAN PATENT SPECIFICATION

(11) **EP 3 294 895 B1**
(45) Date of publication and mention of the grant of the patent: **09.09.2020**
(21) Application number: 16710350.6
(22) Date of filing: 15.02.2016
(51) Int. Cl.: C12N 15/867, A61K 39/12, A61K 48/00, C12N 15/82, C12N 15/113, C12N 15/86

(54) **GENE THERAPEUTIC FOR THE TREATMENT OF HIV AND USES THEREOF**
GENETISCHES THERAPEUTIKUM ZUR BEHANDLUNG VON HIV AND DESSEN VERWENDUNG
PRODUIT DE THÉRAPIE GÉNIQUE POUR LE TRAITEMENT DE VIH ET SON UTILISATION

(30) Priority: 18.05.2015 US 201562163332 P
(43) Date of publication of application: 21.03.2018
(73) Proprietor: Calimmune Inc., Tucson AZ 85711 (US); Calimmune Australia Pty Ltd, Darlinghurst, New South Wales 2010 (AU); St. Vincent's Hospital Sydney, Darlinghurst, New South Wales 2010 (AU); NewSouth Innovations Pty Limited, Sydney, NSW 2052 (AU)
(72) Inventor: SUZUKI, Kazuo, Pymble, New South Wales 2013 (AU); KELLEHER, Anthony Dominic, Bangor, New South Wales 2234 (AU); SYMONDS, Geoffrey Phillip, Rose Bay, New South Wales 2029 (AU); AHLENSTIEL, Chantelle Lisa Evelyn, Ryde, New South Wales 2112 (AU)
(74) Representative: Daniels, Jeffrey Nicholas
(86) International application number: PCT/US2016/017931
(87) International publication number: WO 2016/186708

(56) References cited:
- WO-A1-2016/029275
- WO-A2-2011/008348
- LIU YE ET AL: "A Novel Approach to Block HIV-1 Coreceptor CXCR4 in Non-toxic Manner", MOLECULAR BIOTECHNOLOGY, HUMANA PRESS, INC, US, vol. 56, no. 10, 21 May 2014 (2014-05-21), pages 890-902, XP035385764, ISSN: 1073-6085, DOI: 10.1007/S12033-014-9768-7 [retrieved on 2014-05-21]
- J. E. WALKER ET AL: "Generation of an HIV-1-Resistant Immune System with CD34+ Hematopoietic Stem Cells Transduced with a Triple-Combination Anti-HIV Lentiviral Vector", JOURNAL OF VIROLOGY., vol. 86, no. 10, 15 May 2012 (2012-05-15), pages 5719-5729, XP055271437, US ISSN: 0022-538X, DOI: 10.1128/JVI.06300-11
- JOSEPH S ANDERSON ET AL: "Preintegration HIV-1 Inhibition by a Combination Lentiviral Vector Containing a Chimeric TRIM5[alpha] Protein, a CCR5 shRNA, and a TAR Decoy", MOLECULAR THERAPY, vol. 17, no. 12, 1 December 2009 (2009-12-01), pages 2103-2114, XP055038621, ISSN: 1525-0016, DOI: 10.1038/mt.2009.187
- BRYAN P BURKE ET AL: "Engineering Cellular Resistance to HIV-1 Infection In Vivo Using a Dual Therapeutic Lentiviral Vector", MOLECULAR THERAPY-NUCLEIC ACIDS, vol. 4, no. 4, 14 April 2015 (2015-04-14), page e236, XP055270563, DOI: 10.1038/mtna.2015.10 cited in the application & BURKE BRYAN P; BOYD MAUREEN P; MILLINGTON MICHELLE L; IMPEY HELEN; ZHANG JANE; CARROLL MARIA V; CAMBA-COLON JOANNA; KEECH NAOMI; D: "Engineering Resistance to HIV-1 Infection with a Dual Therapeutic Lentiviral Vector", MOLECULAR THERAPY, vol. 21, no. Suppl. 1, 18 May 2013 (2013-05-18), page S20, XP055270676, GB ISSN: 1525-0016
- ORIT WOLSTEIN ET AL: "Preclinical safety and efficacy of an anti-HIV-1 lentiviral vector containing a short hairpin RNA to CCR5 and the C46 fusion inhibitor", MOLECULAR THERAPY - METHODS & CLINICAL DEVELOPMENT, vol. 1, 12 February 2014 (2014-02-12), page 11, XP055270561, DOI: 10.1038/mtm.2013.11 cited in the application
- KAZUO SUZUKI ET AL: "Promoter Targeting shRNA Suppresses HIV-1 Infection In vivo Through Transcriptional Gene Silencing", MOLECULAR THERAPY-NUCLEIC ACIDS, vol. 2, no. 12, 3 December 2013 (2013-12-03), page e137, XP055270343, DOI: 10.1038/mtna.2013.64 cited in the application
- KAZUO SUZUKI ET AL: "Promoter Targeting RNAs: Unexpected Contributors to the Control of HIV-1 Transcription", MOLECULAR THERAPY-NUCLEIC ACIDS, vol. 4, no. 1, 27 January 2015 (2015-01-27), page e222, XP55270351, DOI: 10.1038/mtna.2014.67
- Nick Ct Schopman ET AL: "Anticipating and blocking HIV-1 escape by second generation antiviral shRNAs", Retrovirology, 1 January 2010 (2010-01-01), page 52, XP55271079, Retrieved from the Internet: URL:http://download.springer.com/static/pd f/124/art%3A10.1186%2F1742-4690-7-52.pdf?o riginUrl=http://retrovirology.biomedcentra l.com/article/10.1186/1742-4690-7-52&token 2=exp=1462786700~acl=/static/pdf/124/art%2 53A10.1186%252F1742-4690-7-52.pdf*~hmac=af 37d34519eca362de70dedab78e7d9e4f7eb38c8458 d5854be2a4 [retrieved on 2016-05-09]
- SCOTT G KITCHEN ET AL: "Stem cell-based anti-HIV gene therapy", VIROLOGY, ELSEVIER, AMSTERDAM, NL, vol. 411, no. 2, 19 December 2010 (2010-12-19), pages 260-272, XP028185460, ISSN: 0042-6822, DOI: 10.1016/J.VIROL.2010.12.039 [retrieved on 2010-12-24]
- CATALINA MÉNDEZ: "Post-transcriptional gene silencing, transcriptional gene silencing and human immunodeficiency virus", WORLD JOURNAL OF VIROLOGY, vol. 4, no. 3, 12 August 2015 (2015-08-12) , pages 219-244, XP055270381, US ISSN: 2220-3249, DOI: 10.5501/wjv.v4.i3.219
- CHANTELLE AHLENSTIEL ET AL: "Novel RNA Duplex Locks HIV-1 in a Latent State via Chromatin-mediated Transcriptional Silencing", MOLECULAR THERAPY-NUCLEIC ACIDS, vol. 4, no. 10, 27 October 2015 (2015-10-27), page e261, XP055270333, DOI: 10.1038/mtna.2015.31
- ANNIE GOSSELIN ET AL: "Peripheral Blood CCR4 + CCR6 + and CXCR3 + CCR6 + CD4 + T Cells Are Highly Permissive to HIV-1 Infection", THE JOURNAL OF IMMUNOLOGY, vol. 184, no. 3, 30 December 2009 (2009-12-30), pages 1604-1616, XP55614748, US ISSN: 0022-1767, DOI: 10.4049/jimmunol.0903058
- PATRICIA MONTEIRO ET AL: "Memory CCR6 + CD4 + T Cells Are Preferential Targets for Productive HIV Type 1 Infection Regardless of Their Expression of Integrin [beta] 7", THE JOURNAL OF IMMUNOLOGY, vol. 186, no. 8, 11 March 2011 (2011-03-11), pages 4618-4630, XP55614754, US ISSN: 0022-1767, DOI: 10.4049/jimmunol.1004151
- C. JIANG ET AL: "Primary Infection by a Human Immunodeficiency Virus with Atypical Coreceptor Tropism", JOURNAL OF VIROLOGY., vol. 85, no. 20, 10 August 2011 (2011-08-10), pages 10669-10681, XP55614759, US ISSN: 0022-538X, DOI: 10.1128/JVI.05249-11

## Description

### FIELD OF DISCLSOURE

This disclosure generally relates to the fields of molecular biology and virology. In particular, the disclosure relates to an expression vector useful in the treatment and prevention of HIV infections as well as a method of inhibiting HIV infection and replication in tissue culture systems.

### BACKGROUND OF THE DISCLSOURE

HIV-1 is the causative agent of Acquired Immunodeficiency Syndrome (AIDS) with of the order of 30 million individuals infected world-wide. HIV causes the immune system to fail and increases the probability of death due to opportunistic infections. HIV infection is a major global health problem as evidenced by its designation as a pandemic by the World Health Organization. Most people who are infected with HIV, particularly in the developing world, eventually develop AIDS, which claims the lives of more than one million people every year.

HIV-1 belongs to the retroviridae family of viruses, and is an enveloped virus whose genome consists of two single stranded RNA molecules (ssRNA). The primary target of HIV-1 is CD4+ expressing cells, such as CD4+ T cells. A glycoprotein of the HIV-1 virus interacts with the CD4 molecule of target cells and with chemokine co- receptors, CCRS or CXCR4 on the surface of target cells. Following fusion and entry into the target cell, the nucleocapsid containing the viral genome dissociates, releasing the contents of the virus, including the ssRNA, into the cytoplasm. A reverse transcriptase (RT) enzyme of HIV-1 synthesizes viral double stranded DNA (dsDNA) from the ssRNA genome. Following synthesis of the double stranded HIV-1 DNA molecule, the HIV-1 DNA is integrated into the host genome.

The integrated HIV-1 DNA is flanked by identical 5' and 3' long terminal repeat sequences (LTR) from which HIV-1 can initiate transcription of the integrated HIV-1 genome. Transcription of the viral DNA requires transcription factors, such as NF-kB, which are upregulated in activated T cells. As a consequence, viral transcription is most active in the T cell following activation of the T cell, such as during infection. Viral RNA resulting from transcription of the integrated HIV-1 genome is subsequently translated and packaged into virus particles which then exit the cell to become infectious virus.

Therapy for HIV-1 infection includes combination antiretroviral therapy (cART). cART, which includes combinations of nucleoside analogue reverse transcriptase inhibitors, protease inhibitors, non-nucleoside reverse transcriptase inhibitors, integrase and fusion inhibitors, slows HIV progression. This, in turn, dramatically decreases the morbidity and mortality rate from HIV/AIDS in regions of the world where the therapy is available. However, cART does not cure or completely eliminate all the symptoms of HIV/AIDS. Also, cART therapy can be compromised by drug resistant mutations, and has a range of side effects which can be serious and which appear to be cumulative. Further, interruption of cART therapy almost invariably leads to the re-emergence of detectable viral replication and the progression to AIDS and has been shown to be associated with an increased incidence of all causes of mortality and serious non AIDS events. For these reasons, as well as the high cost of cART and need for strict adherence, such therapy can be relatively ineffective for a large number of patients.

HIV-based lentiviral vectors are rapidly becoming the retrovirus vector system of choice for research and clinical gene transfer applications. The enhanced ability of lentiviral vectors to transduce both quiescent stem cells and non-dividing terminally differentiated cells has led to the development of a wide range of therapeutic gene delivery vectors, as well as promising research tools, such as short hairpin RNA (shRNA) gene knockdown libraries and vectors for induction of pluripotency in terminally differentiated cells. Early gamma-retroviral clinical gene therapy vectors restored immune function in patients with X-linked severe combined immunodeficiency (SCID-X1), but they were subsequently found to cause proliferative disorders via transactivation of proto-oncogenes. Newer lentiviral vector designs may significantly reduce that risk, and they await clinical testing for final validation of their predicted safety. The field remains in flux and the outcomes of the clinical testing are unpredictable.

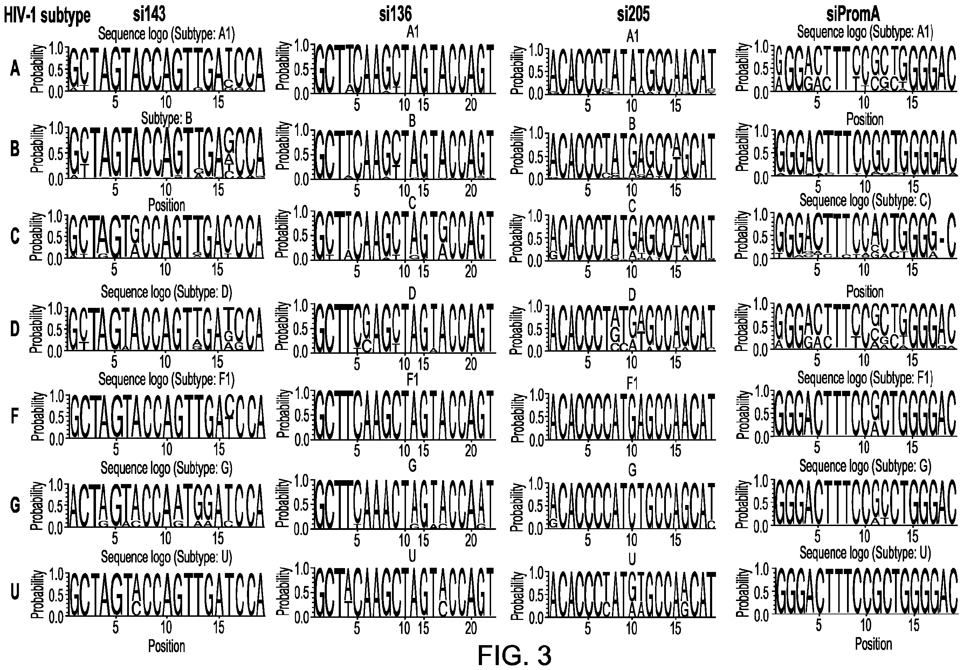

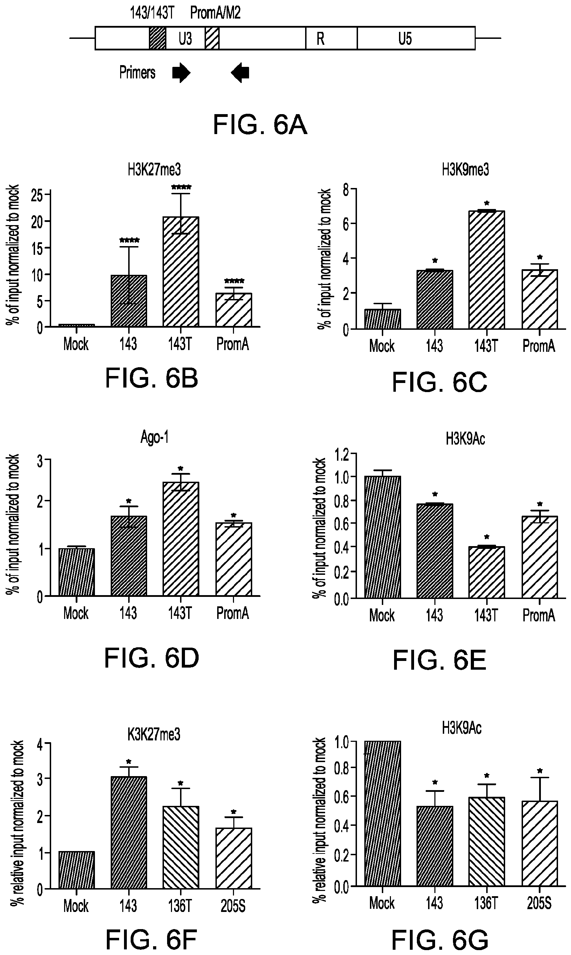

For instance, Trobridge et al., PLoS One, Vol. 4: e7693, 2009 (hereinafter "Trobridge") teach that insertion of a pol III-driven shRNA in the same expression vector as a C46 fusion inhibitor gene driven by a separate promoter resulted in a significant reduction in C46 expression as compared to a vector that did not include the shRNA (see page 5, left column, first paragraph and Figure 3 of Trobridge). Consequently, the dual vector was substantially less efficacious (more than 27-fold less effective) against HIV infection than the vector encoding the C46 fusion inhibitor alone (see Id.). In addition, the dual vector exhibited significantly lower rates of anti-HIV gene transfer to hematopoietic cells as compared to the vector encoding the C46 fusion inhibitor alone (see pages 6 and 7, Table 1, and Figure 6). Trobridge attributed the poor expression of C46 to the inclusion of the shRNA-encoding sequence in the vector (see page 7, right column, first paragraph and page 9, left column, third paragraph). Trobridge also discloses that low gene transfer levels are a major roadblock in clinical trials for anti-HIV gene therapy (see, e.g., abstract, page 2, left column, first full paragraph, and page 8, right column, second full paragraph).

Liu et al., discloses a novel approach to block HIV-1 coreceptor CXCR4 in non-toxic manner (Mol. Biotechnol.; 56(10): 890-902, 21 May 2014). Walker et al., discloses generation of an HIV-1-resistant immune system with CD34+ hematopoietic stem cells transduced with a triple-combination anti-HIV lentiviral vector (J. Virol.; 86(10): 5719-5729, 15 May 2012). Burke et al., discloses engineering cellular resistance to HIV-1 infection in vivo using a dual therapeutic lentiviral vector (Molecular Therapy - Nucleic Acids; 4(4): e236, 14 April 2015. Wolstein et al., discloses preclinical safety and efficacy of an anti-HIV-1 lentiviral vector containing a short hairpin RNA to CCR5 and the C46 fusion inhibitor (Molecular Therapy - Methods & Clinical Development; 1: 11, 12 February 2014). WO 2011/008348 A2 (Calimmune Inc., 20 January 2011) discloses dual vector inhibition of human immunodeficiency virus.

### SUMMARY OF THE DISCLOSURE

The present disclosure provides a novel therapeutic approach for use in treating, preventing and/or reducing HIV infection in which a combination of different steps in viral infection are targeted by gene therapy. In a first aspect of the present invention an expression vector according to claim 1 is provided. In particular, the present invention provides an expression vector comprising (i) at least one nucleic acid sequence encoding a transcriptional gene silencing element; and (ii) at least two other nucleic acid sequences selected from the group consisting of a nucleic acid sequence that encodes a nucleic acid molecule that inhibits an HIV co-receptor; a nucleic acid sequence that encodes an HIV fusion inhibitor protein; a nucleic acid sequence encoding an inhibitor of HIV replication; and a nucleic acid sequence encoding an inhibitor of viral entry, wherein the at least one nucleic acid sequence encoding a transcriptional gene silencing element is selected from the group consisting of (i) a silencing nucleic acid which targets a sequence from about position 143 to about position 161 of the 5' LTR of HIV-1, (ii) a silencing nucleic acid which targets a sequence from about position 136 to about position 154 of the 5' LTR of HIV-1, (iii) a silencing nucleic acid which targets a sequence from about position 205 to about position 223 of the 5' LTR of HIV-1; and (iv) a silencing nucleic acid which targets a sequence from about position 350 to about position 368 of the 5' LTR of HIV-1 (the sequence from position 350 to 368 is also referred to herein as "PromA"). In some embodiments, the at least two other nucleic acid sequences comprise a nucleic acid sequence that encodes a nucleic acid molecule that inhibits an HIV co-receptor; and a nucleic acid sequence that encodes an HIV fusion inhibitor protein. In other aspects, the at least two other nucleic acid sequences comprise a nucleic acid sequence that encodes a nucleic acid molecule that inhibits an HIV co-receptor; and a nucleic acid sequence encoding an inhibitor of HIV replication.

In some aspects, the at least one nucleic acid sequence encoding a transcriptional gene silencing element is selected from the group consisting of (i) a silencing nucleic acid which targets the sequence of SEQ ID NO: 1, (ii) a silencing nucleic acid which targets the sequence of SEQ ID NO: 9, (iii) a silencing nucleic acid which targets the sequence of SEQ ID NO: 17; (iv) a silencing nucleic acid which targets a sequence having at least 95% identity with the sequence of SEQ ID NO: 1; (v) a silencing nucleic acid which targets a sequence having at least 95% identity with the sequence of SEQ ID NO: 9; (vi) a silencing nucleic acid which targets a sequence having at least 95% identity with the sequence of SEQ ID NO: 17; (vii) a silencing nucleic acid which targets the sequence of SEQ ID NO: 36; and (viii) a silencing nucleic acid which targets a sequence having at least 95% identity with a sequence of SEQ ID NO: 36.

In some embodiments, the at least one nucleic acid sequence encoding a transcriptional gene silencing element is an RNA duplex comprising a sense strand and an antisense strand, wherein the sense strand comprises a sequence having at least 95% identity to one of the sequence of SEQ ID NO: 6, the sequence of SEQ ID NO: 14, the sequence of SEQ ID NO: 22; or the sequence of SEQ ID NO: 40. In some aspects, the at least one nucleic acid sequence encoding a transcriptional gene silencing element is an siRNA selected from the group consisting of (i) an siRNA comprising a sense strand having the sequence of SEQ ID NO: 6, and an antisense strand having the sequence of SEQ ID NO: 7; (ii) an siRNA comprising a sense strand having the sequence of SEQ ID NO: 14, and an antisense strand having the sequence of SEQ ID NO: 15; (iii) an siRNA comprising a sense strand having the sequence of SEQ ID NO: 22, and an antisense strand having the sequence of SEQ ID NO: 23; and (iv) an siRNA comprising a sense strand having the sequence of SEQ ID NO: 40, and an antisense strand having the sequence of SEQ ID NO: 41.

In some embodiments, the at least one nucleic acid sequence encoding a transcriptional gene silencing element is an shRNA selected from the group consisting of (i) an shRNA having the sequence of SEQ ID NO: 8; (ii) an shRNA having the sequence of SEQ ID NO: 16; and (iii) an shRNA having the sequence of SEQ ID NO: 24; and (iv) an shRNA having the sequence of SEQ ID NO: 42.

In some aspects, the nucleic acid sequence that encodes a nucleic acid molecule that inhibits an HIV co-receptor is a siRNA or shRNA having a double-stranded region, wherein a first portion of the double-stranded region comprises a sequence that is identical to part of a sequence of the HIV co-receptor, and wherein a second portion of the double-stranded region comprises a sequence that is complementary to another part of the sequence of the HIV co-receptor. In some embodiments, the HIV co-receptor is CCR5 or CXCR4. In some embodiments, the nucleic acid sequence that encodes a nucleic acid molecule that inhibits an HIV co-receptor is a shRNA which has a sequence of SEQ ID NO: 25. In some aspects the inhibitor of the HIV co-receptor is capable of reducing expression of the HIV co-receptor when the vector is expressed in a host cell. In some embodiments, the protein that inhibits HIV fusion to a target cell is a C46 protein. In some embodiments, the protein that inhibits HIV fusion has the sequence of SEQ ID NO: 26. In some embodiments, the protein that inhibits HIV replication is selected from the group consisting of human TRIM5α, rhesus TRIM5α, chimeric TRIM5α, a human TRIM5-cyclophilin fusion protein, cyclophilin, E3 ubiquitin, APOBEC3G, and bone marrow stromal cell antigen 2 (BST-2).

In some embodiments, the expression vector comprises at least two nucleic acids encoding a transcriptional gene silencing element. In some embodiments, the expression vector comprises three nucleic acids encoding a transcriptional gene silencing element. In some embodiments, the expression vector is a viral vector. In some embodiments, the viral vector is a lentiviral vector or a retroviral vector. In some embodiments, the lentiviral vector is self-inactivating. In some embodiments, the expression vector, when expressed in a host cell, confers resistance to infection by (1) X4- and R5-tropic HIV strains, (2) highly active antiretroviral therapy (HAART) resistant HIV strains or (3) X4- and R5-tropic HAART-resistant HIV strains. In some embodiments, each of the nucleic acids are expressed by separate promoters. In some embodiments, least two of the nucleic acids are expressed by a same promoter.

In another embodiment of the present invention the expression vector comprises a first nucleic acid sequence encoding an inhibitory nucleic acid capable of reducing expression of an HIV co-receptor; a second nucleic acid sequence encoding an HIV fusion inhibitor protein; and a third nucleic acid sequence encoding a silencing nucleic acid that targets a sequence of a 5' LTR of HIV selected from the group consisting of (i) a silencing nucleic acid which targets a sequence from about position 143 to about position 161 of the 5' LTR of HIV-1, (ii) a silencing nucleic acid which targets a sequence from about position 136 to about position 154 of the 5' LTR of HIV-1, (iii) a silencing nucleic acid which targets a sequence from about position 205 to about position 223 of the 5' LTR of HIV-1; and (iv) a silencing nucleic acid which targets a sequence from about position 350 to about position 368 of the 5' LTR of HIV. In some embodiments, the first nucleic acid sequence is operably linked to a first promoter; the second nucleic acid sequence is operably linked to a second promoter; and the third nucleic acid sequence is operably linked to a third promoter. In some embodiments, at least two of the first, second, and third promoters are the same. In some embodiments, the first, second, and third promoters are different. In some embodiments, the expression vector further comprises a fourth nucleic acid, wherein the fourth nucleic acid is selected from the group consisting of another nucleic acid that targets a sequence of the 5' LTR of HIV, a nucleic acid sequence encoding an inhibitor of HIV replication; and a nucleic acid sequence encoding an inhibitor of viral entry; wherein the four nucleic acid is operably linked to a fourth promoter. In some embodiments, the expression vector comprises at least two silencing nucleic acids that target the 5'LTR of HIV. In some embodiments, the expression vector is a lentiviral vector.

In another embodiment of the present invention the expression vector comprises a first nucleic acid encoding an shRNA having a sequence of SEQ ID NO: 25; a second nucleic acid sequence encoding for a C46 protein; and a third nucleic acid sequence encoding a silencing nucleic acid that targets one of a sequence of a 5' LTR of HIV selected from the group consisting of (i) a silencing nucleic acid which targets a sequence from about position 143 to about position 161 of the 5' LTR of HIV-1, (ii) a silencing nucleic acid which targets a sequence from about position 136 to about position 154 of the 5' LTR of HIV-1, (iii) a silencing nucleic acid which targets a sequence from about position 205 to about position 223 of the 5' LTR of HIV-1; and (iv) a silencing nucleic acid which targets a sequence from about position 350 to about position 368 of the 5' LTR of HIV-1; wherein the first nucleic acid sequence is operably linked to an H1 pol III promoter, the second nucleic acid sequence is operably linked to a pol II promoters, such as the UbiquitinC pol II promoter, the third nucleic acid sequence operably linked to one of a U6, U6.1, or U6.2 promoter (U6, U6.1, and U6.2 may be collectively referred to as "U6" herein). In some embodiments, the expression vector further comprises a fourth nucleic acid sequence, wherein the fourth nucleic acid sequence is another of a sequence of a 5' LTR of HIV selected from the group consisting of (i) a silencing nucleic acid which targets a sequence from about position 143 to about position 161 of the 5' LTR of HIV-1, (ii) a silencing nucleic acid which targets a sequence from about position 136 to about position 154 of the 5' LTR of HIV-1, (iii) a silencing nucleic acid which targets a sequence from about position 205 to about position 223 of the 5' LTR of HIV-1; and (iv) a silencing nucleic acid which targets a sequence from about position 350 to about position 368 of the 5' LTR of HIV-1; and wherein the fourth nucleic acid sequence is operably linked to a U1 promoter. In some embodiments, the expression vector is a lentiviral vector.

In another embodiment of the present invention the expression vector comprises a first nucleic acid sequence encoding an inhibitory nucleic acid capable of reducing expression of an HIV co-receptor; a second nucleic acid sequence encoding an HIV fusion inhibitor protein, wherein the second nucleic acid is not T20; and a third nucleic acid sequence encoding a silencing nucleic acid that targets a sequence with a 5' LTR region of HIV, wherein the third nucleic acid is selected from the group consisting of (i) a silencing nucleic acid which targets a sequence from about position 143 to about position 161 of the 5' LTR of HIV-1, (ii) a silencing nucleic acid which targets a sequence from about position 136 to about position 154 of the 5' LTR of HIV-1, (iii) a silencing nucleic acid which targets a sequence from about position 205 to about position 223 of the 5' LTR of HIV-1; and (iv) a silencing nucleic acid which targets a sequence from about position 350 to about position 368 of the 5' LTR of HIV-1. In some embodiments, the second nucleic acid sequence encoding an HIV fusion inhibitor protein has the sequence of SEQ ID NO: 26.

In a second aspect the present invention provides a host cell according to claim 10. In one embodiment, the present invention provides a host cell comprising an expression vector comprising a first nucleic acid sequence encoding an inhibitory nucleic acid capable of reducing expression of an HIV co-receptor; a second nucleic acid sequence encoding an HIV fusion inhibitor protein; and a third nucleic acid sequence encoding a silencing nucleic acid that targets a sequence of a 5' LTR of HIV, wherein the third nucleic acid is selected from the group consisting of (i) a silencing nucleic acid which targets a sequence from about position 143 to about position 161 of the 5' LTR of HIV-1, (ii) a silencing nucleic acid which targets a sequence from about position 136 to about position 154 of the 5' LTR of HIV-1, (iii) a silencing nucleic acid which targets a sequence from about position 205 to about position 223 of the 5' LTR of HIV-1; and (iv) a silencing nucleic acid which targets a sequence from about position 350 to about position 368 of the 5' LTR of HIV-1. In some embodiments, the host cell is a hematopoietic progenitor/stem cell, a monocyte, a macrophage, a peripheral blood mononuclear cell, a CD4+ T lymphocyte, a CD8+ T lymphocyte, or a dendritic cell.

In a third aspect the present invention provides a composition according to claim 14, comprising the expression vector of the invention and a pharmaceutically acceptable carrier or excipient. The composition may further comprise another nucleic acid molecule. Thus, an embodiment in accordance with this aspect of the present invention is a composition, including a pharmaceutical composition, comprising an expression vector including a first nucleic acid sequence encoding an inhibitory nucleic acid capable of reducing expression of an HIV co-receptor; a second nucleic acid sequence encoding an HIV fusion inhibitor protein; and a third nucleic acid sequence encoding a silencing nucleic acid that targets a sequence of a 5' LTR of HIV, wherein the third nucleic acid is selected from the group consisting of (i) a silencing nucleic acid which targets a sequence from about position 143 to about position 161 of the 5' LTR of HIV-1, (ii) a silencing nucleic acid which targets a sequence from about position 136 to about position 154 of the 5' LTR of HIV-1, (iii) a silencing nucleic acid which targets a sequence from about position 205 to about position 223 of the 5' LTR of HIV-1; and (iv) a silencing nucleic acid which targets a sequence from about position 350 to about position 368 of the 5' LTR of HIV-1. In some embodiments, the expression vector is a lentiviral vector. In some embodiments, the composition is formulated as an emulsion. In some embodiments, the composition is formulated with micelles, nanoparticles, or nanocapsules. In some embodiments, the compositions are encapsulated within a polymer. In some embodiments, compositions are encapsulated within liposomes. In some embodiments, the compositions are encapsulated within minicells. In some embodiments, the nucleic acid encoding an HIV fusion inhibitor is other than T20.

In another aspect of the present disclosure is a composition comprising (i) an expression vector comprising at least two nucleic acids, wherein the at least two nucleic acids are selected from the group consisting of a nucleic acid sequence that encodes a nucleic acid molecule that inhibits an HIV co-receptor; a nucleic acid sequence that encodes an HIV fusion inhibitor protein; a nucleic acid sequence encoding an inhibitor of HIV replication; and a nucleic acid sequence encoding an inhibitor of viral entry; and (ii) a nucleic acid that encodes at least one transcriptional gene silencing element which targets a sequence of the 5' LTR of HIV, wherein the nucleic acid that encodes at least one transcriptional gene silencing element is selected from the group consisting of (i) a silencing nucleic acid which targets the sequence of SEQ ID NO: 1, (ii) a silencing nucleic acid which targets the sequence of SEQ ID NO: 9, (iii) a silencing nucleic acid which targets the sequence of SEQ ID NO: 17; (iv) a silencing nucleic acid which targets the sequence of SEQ ID NO: 36; (v) a silencing nucleic acid which targets a sequence having at least 95% identity with the sequence of SEQ ID NO: 1; (vi) a silencing nucleic acid which targets a sequence having at least 95% identity with the sequence of SEQ ID NO: 9; (vii) a silencing nucleic acid which targets a sequence having at least 95% identity with the sequence of SEQ ID NO: 17; and (viii) a silencing nucleic acid which targets a sequence having at least 95% identity with the sequence of SEQ ID NO: 36. In some embodiments, the nucleic acid that encodes at least one transcriptional gene silencing element is an RNA duplex comprising a sense strand and an antisense strand, wherein the sense strand comprises a sequence having at least 95% identity to one of the sequence of SEQ ID NO: 6, the sequence of SEQ ID NO: 14, the sequence of SEQ ID NO: 22, or the sequence or SEQ ID NO: 40. In some embodiments, the nucleic acid that encodes at least one transcriptional gene silencing element is an siRNA selected from the group consisting of (i) an siRNA comprising a sense strand having the sequence of SEQ ID NO: 6, and an antisense strand having the sequence of SEQ ID NO: 7; (ii) an siRNA comprising a sense strand having the sequence of SEQ ID NO: 14, and an antisense strand having the sequence of SEQ ID NO: 15; (iii) an siRNA comprising a sense strand having the sequence of SEQ ID NO: 22, and an antisense strand having the sequence of SEQ ID NO: 23; and (iv) an siRNA comprising a sense strand having the sequence of SEQ ID NO: 40, and an antisense strand having the sequence of SEQ ID NO: 41. In some embodiments, the nucleic acid that encodes at least one transcriptional gene silencing element is an shRNA selected from the group consisting of (i) an shRNA having the sequence of SEQ ID NO: 8; (ii) an shRNA having the sequence of SEQ ID NO: 16; (iii) an shRNA having the sequence of SEQ ID NO: 24; (iv) an shRNA having the sequence of SEQ ID NO: 42.

In a fourth aspect the present invention provides an expression vector for use in the treatment of HIV according to claim 12.

In other aspects the present disclosure provides expression vectors for use in methods, including (i) methods of inhibiting HIV transcription; (ii) methods of inhibiting HIV replication; (iii) methods of treating an HIV infection; (iv) methods of preventing an HIV infection; (v) methods of reducing an HIV infection; and (vi) methods of preventing and/or reducing a productive HIV infection in a subject not suffering from any HIV infection. These methods are to be carried out by administering or contacting a cell with an expression vector as described herein, a composition comprising an expression vector and/or other components or active agents, or co-administering a composition comprising (a) an expression vector, and (b) another component or active agent.

In an embodiment the present invention provides an expression vector according to the invention for use in a method of inhibiting HIV gene transcription in a cell infected with HIV comprising contacting the cell with an effective amount of the expression vector comprising a first nucleic acid sequence encoding an inhibitory nucleic acid capable of reducing expression of an HIV co-receptor; a second nucleic acid sequence encoding an HIV fusion inhibitor protein; and a third nucleic acid sequence encoding a silencing nucleic acid that targets a sequence of a 5' LTR of HIV, wherein the third nucleic acid is selected from the group consisting of (i) a silencing nucleic acid which targets a sequence from about position 143 to about position 161 of the 5' LTR of HIV-1, (ii) a silencing nucleic acid which targets a sequence from about position 136 to about position 154 of the 5' LTR of HIV-1, (iii) a silencing nucleic acid which targets a sequence from about position 205 to about position 223 of the 5' LTR of HIV-1; and (iv) a silencing nucleic acid which targets a sequence from about position 350 to about position 368 of the 5' LTR of HIV-1.

Another embodiment of the present invention is an expression vector according to the invention for use in a method of inhibiting HIV gene replication in a cell infected with HIV, comprising contacting the cell with an effective amount of the expression vector comprising a first nucleic acid sequence encoding an inhibitory nucleic acid capable of reducing expression of an HIV co-receptor; a second nucleic acid sequence encoding an HIV fusion inhibitor protein; and a third nucleic acid sequence encoding a silencing nucleic acid that targets a sequence of a 5' LTR of HIV, wherein the third nucleic acid is selected from the group consisting of (i) a silencing nucleic acid which targets a sequence from about position 143 to about position 161 of the 5' LTR of HIV-1, (ii) a silencing nucleic acid which targets a sequence from about position 136 to about position 154 of the 5' LTR of HIV-1, (iii) a silencing nucleic acid which targets a sequence from about position 205 to about position 223 of the 5' LTR of HIV-1; and (iv) a silencing nucleic acid which targets a sequence from about position 350 to about position 368 of the 5' LTR of HIV-1. In some embodiments, the nucleic acid encoding an HIV fusion inhibitor is other than T20.

In another embodiment the present invention provides an expression vector according to the invention for use in a method of treating an HIV infection in a subject, wherein the use comprises administering to the subject an effective amount of the expression vector comprising a first nucleic acid sequence encoding an inhibitory nucleic acid capable of reducing expression of an HIV co-receptor; a second nucleic acid sequence encoding an HIV fusion inhibitor protein; and a third nucleic acid sequence encoding a silencing nucleic acid that targets a sequence of a 5' LTR of HIV, wherein the third nucleic acid is selected from the group consisting of (i) a silencing nucleic acid which targets a sequence from about position 143 to about position 161 of the 5' LTR of HIV-1, (ii) a silencing nucleic acid which targets a sequence from about position 136 to about position 154 of the 5' LTR of HIV-1, (iii) a silencing nucleic acid which targets a sequence from about position 205 to about position 223 of the 5' LTR of HIV-1; and (iv) a silencing nucleic acid which targets a sequence from about position 350 to about position 368 of the 5' LTR of HIV-1. In some embodiments, the nucleic acid encoding an HIV fusion inhibitor is other than T20.

In another embodiment the present invention provides an expression vector according to the invention for use in a method of preventing and/or reducing an HIV infection in a subject, wherein the use comprises administering to the subject an effective amount of the expression vector comprising a first nucleic acid sequence encoding an inhibitory nucleic acid capable of reducing expression of an HIV co-receptor; a second nucleic acid sequence encoding an HIV fusion inhibitor protein; and a third nucleic acid sequence encoding a silencing nucleic acid that targets a sequence of a 5' LTR of HIV, wherein the third nucleic acid is selected from the group consisting of (i) a silencing nucleic acid which targets a sequence from about position 143 to about position 161 of the 5' LTR of HIV-1, (ii) a silencing nucleic acid which targets a sequence from about position 136 to about position 154 of the 5' LTR of HIV-1, (iii) a silencing nucleic acid which targets a sequence from about position 205 to about position 223 of the 5' LTR of HIV-1; and (iv) a silencing nucleic acid which targets a sequence from about position 350 to about position 368 of the 5' LTR of HIV-1. In some embodiments, the nucleic acid encoding an HIV fusion inhibitor is other than T20.

In another aspect the present invention provides an expression vector for use in a method of preventing and/or reducing a productive HIV infection in a subject not suffering from an HIV infection, comprising administering to the subject an effective amount of the expression vector comprising a first nucleic acid sequence encoding an inhibitory nucleic acid capable of reducing expression of an HIV co-receptor; a second nucleic acid sequence encoding an HIV fusion inhibitor protein; and a third nucleic acid sequence encoding a silencing nucleic acid that targets a sequence of a 5' LTR of HIV, wherein the third nucleic acid is selected from the group consisting of (i) a silencing nucleic acid which targets a sequence from about position 143 to about position 161 of the 5' LTR of HIV-1, (ii) a silencing nucleic acid which targets a sequence from about position 136 to about position 154 of the 5' LTR of HIV-1, (iii) a silencing nucleic acid which targets a sequence from about position 205 to about position 223 of the 5' LTR of HIV-1; and (iv) a silencing nucleic acid which targets a sequence from about position 350 to about position 368 of the 5' LTR of HIV-1. In some embodiments, the nucleic acid encoding an HIV fusion inhibitor is other than T20.

In another aspect the present disclosure provides an expression vector for use in a method of inhibiting HIV gene transcription in a cell infected with HIV, comprising contacting the cell with an effective amount of a composition comprising (i) an expression vector comprising at least two nucleic acids, wherein the at least two nucleic acids are selected from the group consisting of a nucleic acid sequence that encodes a nucleic acid molecule that inhibits an HIV co-receptor; a nucleic acid sequence that encodes an HIV fusion inhibitor protein; a nucleic acid sequence encoding an inhibitor of HIV replication; and a nucleic acid sequence encoding an inhibitor of viral entry; and (ii) a nucleic acid that encodes at least one transcriptional gene silencing element which targets a sequence of the 5' LTR of HIV.

In another aspect the present disclosure provides an expression vector for use in a method of inhibiting HIV gene replication in a cell infected with HIV, comprising contacting the cell with an effective amount of a composition comprising (i) an expression vector comprising at least two nucleic acids, wherein the at least two nucleic acids are selected from the group consisting of a nucleic acid sequence that encodes a nucleic acid molecule that inhibits an HIV co-receptor; a nucleic acid sequence that encodes an HIV fusion inhibitor protein; a nucleic acid sequence encoding an inhibitor of HIV replication; and a nucleic acid sequence encoding an inhibitor of viral entry; and (ii) a nucleic acid that encodes at least one transcriptional gene silencing element which targets a sequence of the 5' LTR of HIV. In some examples, the nucleic acid encoding an HIV fusion inhibitor is other than T20.

In another aspect the present disclosure provides an expression vector for use in a method of treating an HIV infection in a subject, wherein the use comprises administering to the subject an effective amount of a composition comprising (i) an expression vector comprising at least two nucleic acids, wherein the at least two nucleic acids are selected from the group consisting of a nucleic acid sequence that encodes a nucleic acid molecule that inhibits an HIV co-receptor; a nucleic acid sequence that encodes an HIV fusion inhibitor protein; a nucleic acid sequence encoding an inhibitor of HIV replication; and a nucleic acid sequence encoding an inhibitor of viral entry; and (ii) a nucleic acid that encodes at least one transcriptional gene silencing element which targets a sequence of the 5' LTR of HIV.

In another aspect the present disclosure provides an expression vector for use in a method of reducing and/or preventing an HIV infection in a subject, comprising administering to the subject an effective amount of a composition comprising (i) an expression vector comprising at least two nucleic acids, wherein the at least two nucleic acids are selected from the group consisting of a nucleic acid sequence that encodes a nucleic acid molecule that inhibits an HIV co-receptor; a nucleic acid sequence that encodes an HIV fusion inhibitor protein; a nucleic acid sequence encoding an inhibitor of HIV replication; and a nucleic acid sequence encoding an inhibitor of viral entry; and (ii) a nucleic acid that encodes at least one transcriptional gene silencing element which targets a sequence of the 5' LTR of HIV.

In another aspect the present disclosure provides an expression vector for use in a method of preventing and/or reducing a productive HIV infection in a subject not suffering from an HIV infection, comprising administering to the subject an effective amount of a composition comprising (i) an expression vector comprising at least two nucleic acids, wherein the at least two nucleic acids are selected from the group consisting of a nucleic acid sequence that encodes a nucleic acid molecule that inhibits an HIV co-receptor; a nucleic acid sequence that encodes an HIV fusion inhibitor protein; a nucleic acid sequence encoding an inhibitor of HIV replication; and a nucleic acid sequence encoding an inhibitor of viral entry; and (ii) a nucleic acid that encodes at least one transcriptional gene silencing element which targets a sequence of the 5' LTR of HIV.

In another aspect the present disclosure provides an expression vector for use in a method of treating an HIV infection in a subject wherein the use comprises co-administering (i) an effective amount of an expression vector comprising at least two nucleic acids, wherein the at least two nucleic acids are selected from the group consisting of a nucleic acid sequence that encodes a nucleic acid molecule that inhibits an HIV co-receptor; a nucleic acid sequence that encodes an HIV fusion inhibitor protein; a nucleic acid sequence encoding an inhibitor of HIV replication; and a nucleic acid sequence encoding an inhibitor of viral entry; and (ii) an effective amount of at least one silencing nucleic acid selected from the group consisting of (a) a silencing nucleic acid which targets the sequence of SEQ ID NO: 1, (b) a silencing nucleic acid which targets the sequence of SEQ ID NO: 9, (c) a silencing nucleic acid which targets the sequence of SEQ ID NO: 17; (d) a silencing nucleic acid which targets the sequence of SEQ ID NO: 36; (e) a silencing nucleic acid which targets a sequence having at least 95% identity with the sequence of SEQ ID NO: 1; (f) a silencing nucleic acid which targets a sequence having at least 95% identity with the sequence of SEQ ID NO: 9; and (g) a silencing nucleic acid which targets a sequence having at least 95% identity with the sequence of SEQ ID NO: 17; (h) a silencing nucleic acid which targets a sequence having at least 95% identity with the sequence of SEQ ID NO: 36. In some examples, the expression vector is a lentiviral vector. In some examples, the co-administering is simultaneous. In some examples, the expression vector and the at least one transcriptional gene silencing element are administered at different times. In some examples, the expression vector comprises a nucleic acid sequence that encodes a nucleic acid molecule that inhibits an HIV co-receptor; and a nucleic acid sequence that encodes an HIV fusion inhibitor protein. In some examples, the HIV co-receptor is CCR5 and the HIV fusion inhibitor protein is C46. In some examples, the nucleic acid encoding an HIV fusion inhibitor is other than T20.

In another aspect the present disclosure provides an expression vector for use in a method of reducing and/or preventing an HIV infection in a subject comprising co-administering (i) an effective amount of an expression vector comprising at least two nucleic acids, wherein the at least two nucleic acids are selected from the group consisting of a nucleic acid sequence that encodes a nucleic acid molecule that inhibits an HIV co-receptor; a nucleic acid sequence that encodes an HIV fusion inhibitor protein; a nucleic acid sequence encoding an inhibitor of HIV replication; and a nucleic acid sequence encoding an inhibitor of viral entry; and (ii) an effective amount of at least one silencing nucleic acid selected from the group consisting of (a) a silencing nucleic acid which targets the sequence of SEQ ID NO: 1, (b) a silencing nucleic acid which targets the sequence of SEQ ID NO: 9, (c) a silencing nucleic acid which targets the sequence of SEQ ID NO: 17; (d) a silencing nucleic acid which targets the sequence of SEQ ID NO: 36; (e) a silencing nucleic acid which targets a sequence having at least 95% identity with the sequence of SEQ ID NO: 1; (f) a silencing nucleic acid which targets a sequence having at least 95% identity with the sequence of SEQ ID NO: 9; and (g) a silencing nucleic acid which targets a sequence having at least 95% identity with the sequence of SEQ ID NO: 17; (h) a silencing nucleic acid which targets a sequence having at least 95% identity with the sequence of SEQ ID NO: 36. In some examples, the expression vector is a lentiviral vector. In some examples, the co-administering is simultaneous. In some examples, the expression vector and the at least one transcriptional gene silencing element are administered at different times. In some examples, the expression vector comprises a nucleic acid sequence that encodes a nucleic acid molecule that inhibits an HIV co-receptor; and a nucleic acid sequence that encodes an HIV fusion inhibitor protein. In some examples, the HIV co-receptor is CCR5 and the HIV fusion inhibitor protein is C46. In some examples, the nucleic acid encoding an HIV fusion inhibitor is other than T20.

In a fifth aspect the present invention provides an in vitro method for treating human hematopoietic stem cells infected with HIV according to claim 11. In a particular embodiment the method comprises transducing hematopoietic cells of a subject in vitro with the expression vector of the invention, wherein the transduced hematopoietic cells are to be transplanted into the subject, wherein the transduced hematopoietic cells are resistant to HIV infection, wherein the expression vector comprises a first nucleic acid sequence encoding an inhibitory nucleic acid capable of reducing expression of an HIV co-receptor; a second nucleic acid sequence encoding an HIV fusion inhibitor protein; and a third nucleic acid sequence encoding a silencing nucleic acid that targets a sequence of a 5' LTR of HIV selected from the group consisting of (i) a silencing nucleic acid which targets a sequence from about position 143 to about position 161 of the 5' LTR of HIV-1, (ii) a silencing nucleic acid which targets a sequence from about position 136 to about position 154 of the 5' LTR of HIV-1, (iii) a silencing nucleic acid which targets a sequence from about position 205 to about position 223 of the 5' LTR of HIV-1; and (iv) a silencing nucleic acid which targets a sequence from about position 350 to about position 368 of the 5' LTR of HIV-1. The hematopoietic cells are e.g., HPSC, CD4+ T lymphocytes, CD8+ T lymphocytes, or monocyte/macrophages. In some embodiments, the hematopoietic cells are hematopoietic progenitor/stem cells (HPSC) that generate granulocytes, monocyte/macrophages, and lymphocytes that are resistant to HIV infection following transplantation into a patient. In some embodiments, the HPSC are autologous and CD34 positive. In some embodiments, the transduced HPSC can generate granulocytes, monocyte/macrophages, and lymphocytes that are resistant to infection by R5 and X4 tropic strains of HIV. In some embodiments, the transduced HPSC can generate granulocytes, monocyte/macrophages, and lymphocytes that are resistant to infection by HIV strains that are resistant to cART.

In another embodiment of this aspect the present invention provides an in vitro method of reducing an HIV infection in a subject comprising transducing hematopoietic cells with the expression vector of the invention, wherein the transduced hematopoietic cells are to be transplanted into the subject, wherein the transduced hematopoietic cells are resistant to HIV infection, wherein the expression vector comprises a first nucleic acid sequence encoding an inhibitory nucleic acid capable of reducing expression of an HIV co-receptor; a second nucleic acid sequence encoding an HIV fusion inhibitor protein; and a third nucleic acid sequence encoding a silencing nucleic acid that targets a sequence of a 5' LTR of HIV selected from the group consisting of (i) a silencing nucleic acid which targets a sequence from about position 143 to about position 161 of the 5' LTR of HIV-1, (ii) a silencing nucleic acid which targets a sequence from about position 136 to about position 154 of the 5' LTR of HIV-1, (iii) a silencing nucleic acid which targets a sequence from about position 205 to about position 223 of the 5' LTR of HIV-1; and (iv) a silencing nucleic acid which targets a sequence from about position 350 to about position 368 of the 5' LTR of HIV-1. The hematopoietic cells are e.g., HPSC, CD4+ T lymphocytes, CD8+ T lymphocytes, or monocyte/macrophages. In some embodiments, the hematopoietic cells are hematopoietic progenitor/stem cells (HPSC) that generate granulocytes, monocyte/macrophages, and lymphocytes that are resistant to HIV infection following transplantation into a patient. In some embodiments, the HPSC are autologous and CD34 positive. In some embodiments, the transduced HPSC can generate granulocytes, monocyte/macrophages, and lymphocytes that are resistant to infection by R5 and X4 tropic strains of HIV. In some embodiments, the transduced HPSC can generate granulocytes, monocyte/macrophages, and lymphocytes that are resistant to infection by HIV strains that are resistant to cART. In some embodiments, the nucleic acid encoding an HIV fusion inhibitor is other than T20.

In another aspect the present disclosure provides a method of making the expression vectors described herein as well as pharmaceutical compositions comprising the novel expression vectors. In some examples, the method of producing a viral expression vector which, when present in a cell, is capable of inhibiting binding of HIV to the cell and preventing HIV fusion into the cell or HIV replication, comprises synthesizing a cDNA of a gene which expresses a protein capable of preventing HIV fusion into a cell or HIV replication; cloning the synthesized cDNA into a restriction site in a viral vector; and inserting an expression unit capable of down regulating expression of an HIV co-receptor into a restriction site in the vector.

Yet another aspect of the disclosure provides for a kit comprising an expression vector of the present disclosure. The kit may be combined with another active pharmaceutical ingredient for administration to a subject in need thereof.

The present disclosure is based, in part, on the recognition that a therapeutic approach that targets non-HIV genes and/or proteins (i.e. host cell genes and/or proteins) decreases the probability that new HIV strains resistant to the inhibitors will emerge. In untreated CD34+ hematopoietic stem cells (HPSC) and/or CD4+ T cells, HIV binds to CD4 and CCR5 and then fuses with the cell membrane. Following fusion, the viral RNA is introduced and, subsequent to reverse transcription to cDNA that becomes double stranded, it integrates into the host cell genome where it is transcribed to produce more HIV. In the (combination) treated CD34+ HSC and/or CD4+ T cells, HIV is blocked from binding to CCR5 and from fusion with the cell membrane. Without wishing to be bound by any particular theory, it is believed that if HIV overcomes these two blocks and viral RNA is introduced and integrates into the host cell genome as DNA, transcription is blocked as the transcriptional gene silencing (TGS)-inducing shRNA, included within the expression vectors of the present disclosure, causes the HIV DNA to be transcriptionally inactive.

Moreover, and in view of the teachings of Trobridge, the ordinary-skilled artisan would have been led away from including a nucleic acid sequence encoding a fusion inhibitor protein in the same vector as a nucleic acid sequence encoding an inhibitory nucleic acid molecule, let alone including a third nucleic acid sequence encoding a transcriptional gene silencing element. The skilled person would have been led away simply because Trobridge explicitly indicates that dual combination vectors were actually less effective in inhibiting HIV replication than vectors encoding the fusion inhibitor protein alone. There, the very low levels of gene transfer to hematopoietic cells exhibited by the dual vector would have also discouraged one of ordinary skill in the art from modifying a vector encoding a single nucleic acid to incorporate additional nucleic acid sequences because such low gene transfer levels have been the primary reason for failure of gene therapies in clinical trials. Given this, the skilled artisan reading Trobridge would certainly have expected a triple combination vector or a quadruple combination vector, such as disclosed herein, to not work and certainly be less efficacious than any single vector or dual vector systems.

Applicants submit that the present disclosure provides for superior results as compared with Trobridge. In fact, Applicants have demonstrated the ability to stably introduce LVsh5/C46 (also referred to herein as "Cal-1") into various hematopoietic cells, including CD4+ T lymphocytes and CD34+ HSPC, to allow for expression of a CCR5-targeted shRNA (sh5) and C46. In fact, Applicants have demonstrated that treatment of target cells with the LVsh5/C46 vector did not show any indication of toxicity. (See, for example, Wolstein et al., "Preclinical Safety and Efficacy Of An Anti-HIV-1 Lentiviral Vector Containing A Short Hairpin RNA To CCR5 and The C46 Fusion Inhibitor," Molecular Therapy-Methods & Clinical Development (2014) 1, 11; doi:10.1038/mtm.2013.11; see also Burke, et al. "Engineering Cellular Resistance To HIV-1 Infection In Vivo Using A Dual Therapeutic Lentiviral Vector," Molecular Therapy-Nucleic Acids (2015) 4, e236; doi:10.1038/mtna.2015.10).

And, as further noted herein, highly active antiretroviral therapy (e.g. cART) uses two to three active pharmaceutical ingredients to effectively suppress HIV replication and mitigate the development of resistance. Applicants believe that a triple or quadruple combination vector, such as disclosed herein, may likewise provide an efficacious means for treatment and/or suppression of HIV. Accordingly, and without wishing to be bound by any particular theory, Applicants believe that a triple or quadruple combination vector may be as safe as dual vector constructs, while providing comparatively superior results.

### BRIEF DESCRIPTION OF THE DRAWINGS

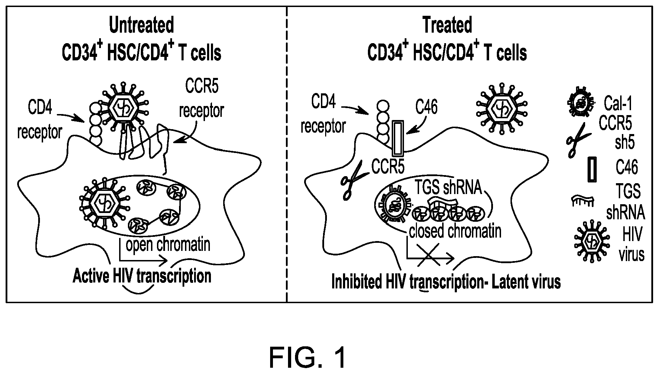

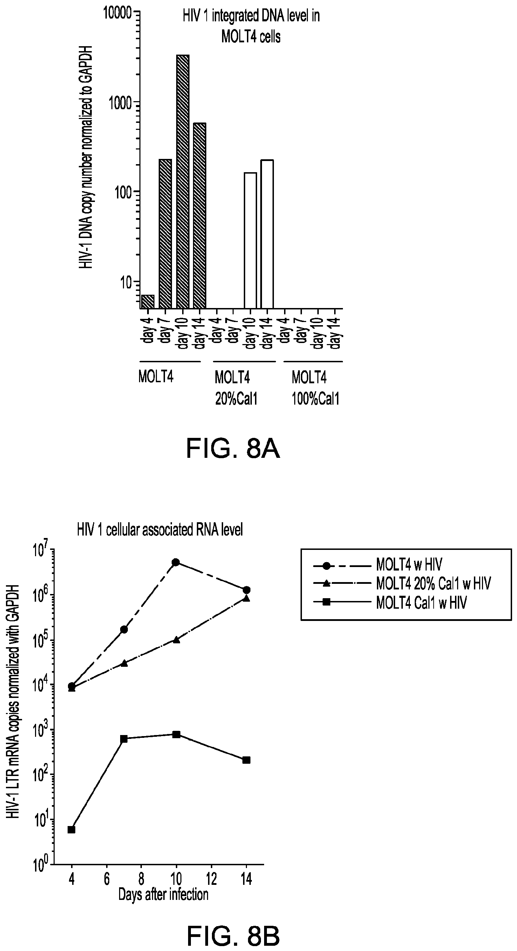

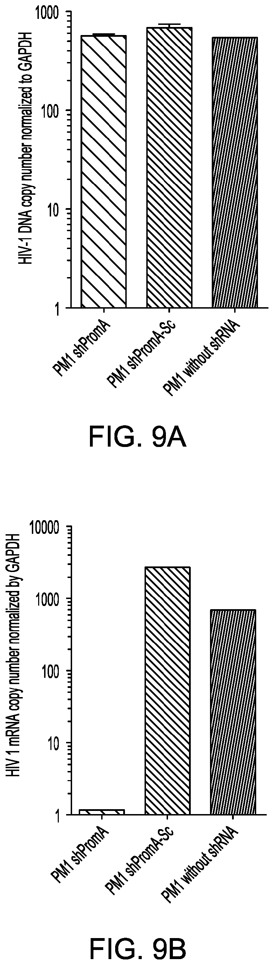

Figure 1 illustrates how the novel combination vector of the present disclosure may inhibit HIV. Model of mechanisms targeted by complex LV construct in protection against HIV infection.

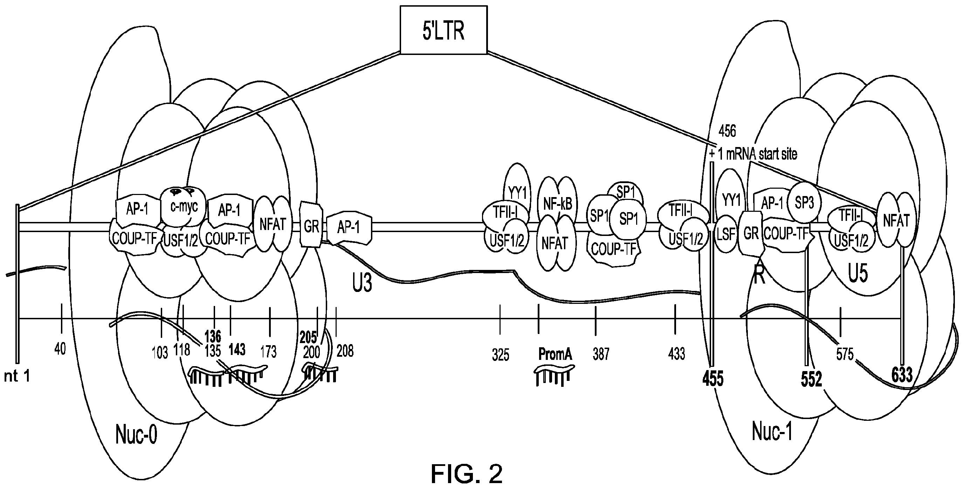

Figure 2 illustrates the position of the TGS-inducing si/shRNAs. Model of the 5'LTR including positions of Nuc-0 and Nuc-1, and target sequences of our lead candidate si/shRNAs. These target the sequence within the tandem NF-kB binding sites (PromA) and a sequence upstream (si143), in the region of nuc-0. These induce similar epigenetic changes. We have shown these molecules act in the nucleus through Agol recruitment followed by HDAC 1 and 2s and HMTs e.g. Enhancer of Zeste.
Figure 3 shows siRNA 143, 136, 205 and PromA target sequence conservation in the HIV-1 5'LTR promoter across subtypes A-U.

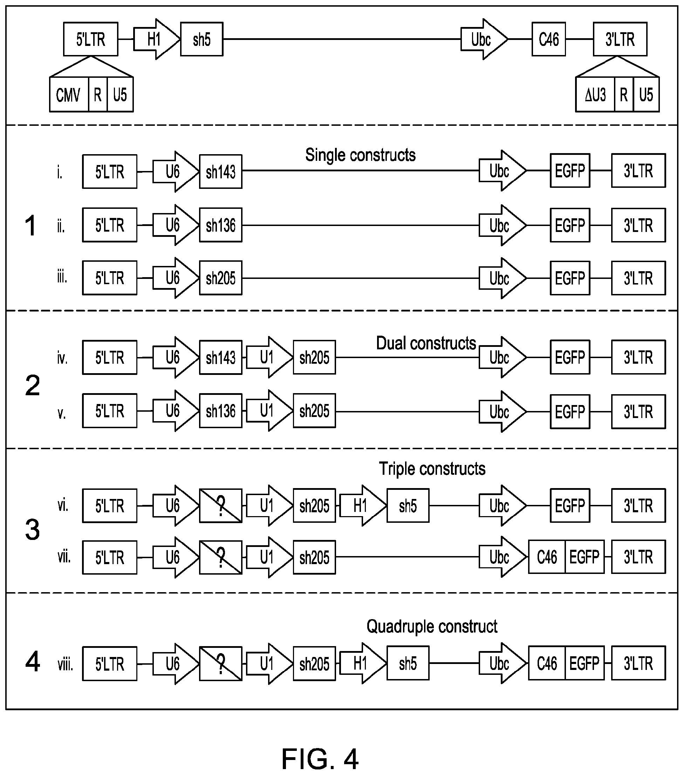

Figure 4 shows self-inactivating lentiviral vectors containing modified LTRs allowing integration but not expression of viral genome, based on the Calimmune Cal-1 backbone vector (see US Publication No. US2012/0201794), consisting of HI, H1 promoter and/or U6, U6 promoter and/or U1, U1 promoter; sh5, short hairpin RNA CCR5; or shPrA, short hairpin RNA PromA; and/or sh143, short hairpin RNA 143; UbC, ubiquitin C promoter; and C46, C46 fusion inhibitor. To measure construct expression all modified constructs contain EGFP, enhanced green fluorescent protein.
   Results for TGS-inducing shRNA. These results are presented as a series of Figures.

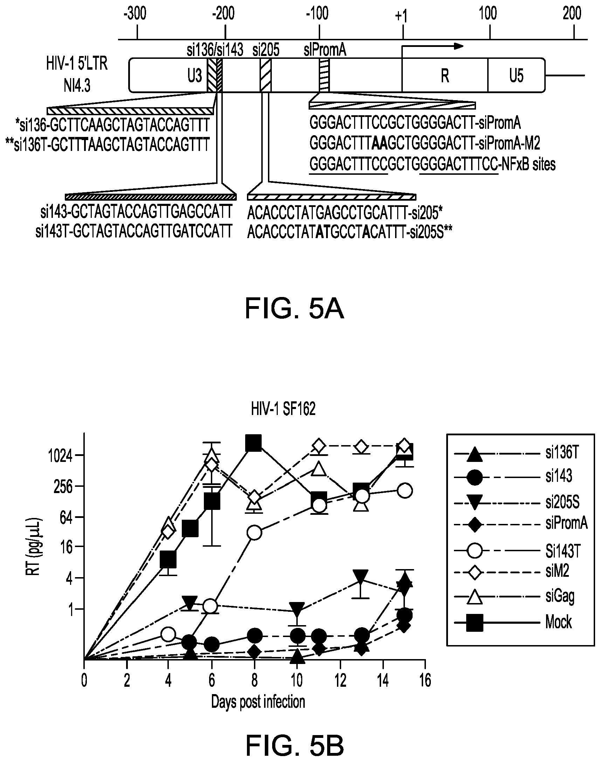

Figure 5a is a diagram showing the region within HIV-1 5'LTR targeted by the 143, 136, 205 and PromA siRNAs. Arrow indicates transcription start site, underlined sequence indicates tandem repeat NF-κB binding sites, siPromA-M2 and si143T are inactive mutants, used as specificity controls. Figure 5b is a graph Si136T-, si143-, si205S- and siPromA-transfected cultures show potent suppression of HIV-1SF162 virus transcription 15 days post-infection compared to inactive mutant controls. * siRNA target identical to HIV-1NL4.3 strain. ** siRNA target identical to HIV-1SF162 strain.
Overall, Figures 5a-b show HIV LTR targeted siRNAs potently suppress HIV transcription. To demonstrate the siRNAs targeting the HIV 5'LTR were capable of potently suppressing HIV-1 transcription, HeLa T4+ cells were transfected with the individual siRNAs, infected with HIV-1 SF162 strain and reverse transcriptase (RT) assays were performed over a prolonged time course of infection. The siRNA 143 target sequence is identical to the SF162 strain. To determine whether a single mismatch is critical in reducing the suppressive effect, we also included siRNA 143T, which has a single T mismatch compared to the SF162 strain. This mismatched siRNA 143T corresponds to the SF162 3'LTR sequence available in GenBank (accession number M65024.1). Also included was the sequence specificity control siPromA-M2. Both of the completely matched siRNAs, 143 and PromA, potently suppressed productive infection with HIV-1 strain SF162, with greater than 1000-fold reduction in RT activity compared to mock-transfected cells. Interestingly, 143T suppressed virus infection to a level comparable to 143 and PromA up to day 6 post-infection, but was then unable to maintain virus suppression.
Figure 6 shows heterochromatin marks observed in siRNA suppressed HIV-1 cultures. To confirm the suppressive effect of the LTR targeting siRNAs was occurring through transcriptional gene silencing (TGS) mechanisms, chromatin immunoprecipitation (ChIP) assays were performed in HeLa T4+ cultures with suppressed HIV-1 following siRNA 143, 136, 205 and PromA transfection or cultures with active HIV transcription following mock-transfection. Heterochromatin marks consisted with TGS were observed at day 6 post-infection in siRNA 143, 136, 205 and PromA-transfected cultures, which included enrichment of histone 3 lysine 27 trimethylation (H3K27me3), and reduction in histone 3 lysine 9 acetylation (H3K9Ac), as compared to mock-transfected cultures. ChIP analysis of heterochromatin marks also showed enrichment of H3K9me3 and recruitment of Agol in si143- and siPromA-transfeced cells.
Heterochromatin marks observed in siRNA suppressed HIV-1 cultures as shown in Figure 6. ChIP analysis of heterochromatin marks showed enrichment of H3K27me3 and reduction of H3K9Ac in si143- and siPromA-transfected cells.

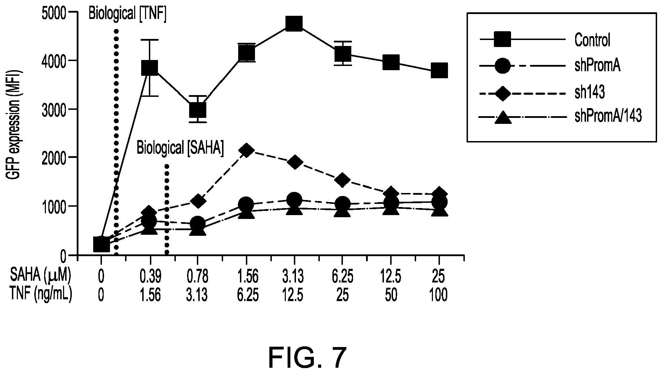

Figure 7 shows robust resistance to reactivation stimuli by shRNA. To investigate the effect of the siRNA 143 target sequence in an HIV-1 latency model, J-Lat 9.2 cells were stably transduced with short hairpin (sh)143 and/or shPromA using lentivirus vectors. Reactivation of integrated latent HIV-1 was attempted in J-Lat 9.2 cells using SAHA, TNF or a combination of SAHA/TNF at various concentrations and GFP expression was measured as a read out of HIV transcription, at 48 h. J-Lat 9.2 cells transduced with sh143 and/or shPromA were observed to be largely resistant to reactivation from SAHA, TNF or combinations of SAHA/TNF at physiological concentrations (Cillo et al., 2014 PNAS; De Pablo-Bernal et al., 2014 J Antimicrob Chemother) and showed low level reactivation even at supra-physiological drug concentrations, while those transduced with a shRNA control showed highly elevated GFP expression, indicating reactivation of latent HIV-1 infection. Importantly, the dual sh143/shPromA transduced J-Lat 9.2 cell line appeared to show slightly better protection against reactivation stimuli. These data demonstrate that the HIV-1 latency model J-Lat 9.2 cells are largely resistant to reactivation by drug treatments when transduced with sh143 and/or shPromA. These findings are important in the context of gene therapy applications, where patients could be exposed to various immune and inflammatory stimuli.
Robust resistance to reactivation stimuli by shRNA as shown in Figure 7. J-Lat 9.2 cells transduced with sh143 and shPromA are less susceptible to reactivation by combined TNF/SAHA treatments, particularly at physiological drug concentrations, compared to control cells, as shown by GFP expression which increases upon reactivation.
Analysis of intracellular HIV-1 DNA is shown in Figure 8a.
Analysis of HIV-1 specific LTR mRNA is shown in Figure 8b.
Analysis of HIV-1 integrated DNA in the PM-1 cells is shown in Figure 9a.
Analysis of HIV-1 specific gag mRNA is shown in Figure 9b.

### DETAILED DESCRIPTION

In general, the present disclosure provides expression vectors, compositions comprising expression vectors, and expression vectors for use in methods of treating subjects by administering such expression vectors or compositions.

As used herein, the singular terms "a," "an," and "the" include plural referents unless the context clearly indicates otherwise. Similarly, the word "or" is intended to include "and" unless the context clearly indicates otherwise.

The terms "comprising," "including," "having," and the like are used interchangeably and have the same meaning. Similarly, "comprises," "includes," "has," and the like are used interchangeably and have the same meaning. Specifically, each of the terms is defined consistent with the common United States patent law definition of "comprising" and is therefore interpreted to be an open term meaning "at least the following," and is also interpreted not to exclude additional features, limitations, aspects, etc. Thus, for example, "a device having components a, b, and c" means that the device includes at least components a, b and c. Similarly, the phrase: "a method involving steps a, b, and c" means that the method includes at least steps a, b, and c. Moreover, while the steps and processes may be outlined herein in a particular order, the skilled artisan will recognize that the ordering steps and processes may vary.

As used herein, a "cell infected with HIV" refers to a cell in which the HIV genome has integrated into the cell genome, and includes cells producing HIV virus, and cells latently infected with HIV. As used herein, a cell latently infected with HIV is a cell in which the HIV genome is integrated into the host cell genome, but which is transcriptionally inactive but capable of reactivation to a transcriptionally active state.

As used herein, "contacting the cell" refers to bringing a composition, formulation, or agent into contact with a cell in a manner which produces an effective result (i.e. reducing, mitigating, or eliminating HIV infection, transcription, or replication). Contacting the cell also includes introducing a composition, formulation, or agent into a cell.

As used herein, the phrases "inhibiting HIV transcription" refers to reducing or preventing transcription of one or more HIV genes to the extent that infectious HIV virus particle formation in the cell is reduced, mitigated, or eliminated.

As used herein, the phrases "inhibiting HIV replication" refers to reducing or preventing replication of one or more HIV genes to the extent that infectious HIV virus particle formation in the cell is reduced, mitigated, or eliminated.

As used herein, the term "HIV" includes not only HIV-1, but also the various strains of HIV-1 (e.g. strain BaL or strain SF162) and the various subtypes of HIV-1 (e.g. subtypes A, B, C, D, F, G H, J, and K).

As used herein, "polynucleotide" refers to single or double stranded DNA, RNA, or modified versions thereof including peptide nucleic acid and locked nucleic acid (LNA).

As used herein, a "silencing nucleic acid" refers to any polynucleotide which is capable of interacting with a specific sequence to inhibit gene expression. Examples of silencing nucleic acids include RNA duplexes (e.g. siRNA, shRNA), LNAs, antisense RNA, DNA polynucleotides which encode sense and/or antisense sequences of the siRNA or shRNA, DNAzymse, or ribozymes. The inhibition of gene expression need not necessarily be gene expression from a specific enumerated sequence, and may be, for example, gene expression from a sequence controlled by that specific sequence.

As used herein "transcriptional gene silencing elements" are silencing nucleic acid sequences which target a sequence within the region of the 5' long terminal repeat (LTR) of HIV (including HIV-1).

### Expression Vectors

The present disclosure provides expression vectors comprising at least three nucleic acid sequences, where the at least three nucleic acids are selected from the group consisting of nucleic acid sequences encoding transcriptional gene silencing elements which target a sequence of the 5' LTR of HIV; nucleic acid sequences that encode inhibitors of an HIV co-receptor; nucleic acid sequences that encode proteins that inhibit HIV fusion to a target cell; nucleic acid sequences that encode proteins that inhibit HIV replication; and nucleic acid sequences that encode an inhibitor of viral entry. As used herein, "expression vector" or "vector" refers to a composition of matter which can be used to deliver nucleic acids of interest to the interior of a cell such that they will be expressed by the cell. Numerous vectors are known in the art including, but not limited to, linear polynucleotides, polynucleotides associated with ionic or amphiphilic compounds, plasmids, and viral vectors. Examples of viral vectors include, but are not limited to, adenoviral vectors, adeno-associated virus vectors, retroviral vectors (including lentiviral vectors), and the like. In one embodiment, the expression vector is a viral vector. Preferably, the viral vector is a retroviral or lentiviral vector.

In embodiments the expression vector comprises at least first, second, and third nucleic acid sequences, wherein the first nucleic acid sequence comprises a nucleic acid sequence encoding a transcriptional gene silencing element as recited in claim 1; wherein the second and third nucleic acid sequences are selected from the group consisting of a nucleic acid sequence that encodes an inhibitor of an HIV co-receptor, a nucleic acid sequence that encodes a protein that inhibits HIV fusion to a target cell, a nucleic acid sequence that encodes a protein that inhibits HIV replication, and a nucleic acid sequence that encodes an inhibitor of viral entry; and wherein the second and third nucleic acid sequences are different.

Yet other embodiments are expression vectors comprising (i) at least one nucleic acid sequence encoding a transcriptional gene silencing element as recited in claim 1; and (ii) at least two other nucleic acid sequences selected from the group consisting of a nucleic acid sequence that encodes an inhibitor of an HIV co-receptor; a nucleic acid sequence that encodes a protein that inhibits HIV fusion to a target cell; a nucleic acid sequence that encodes a protein that inhibits HIV replication; and a nucleic acid sequence that encodes an inhibitor of viral entry. In some embodiments, each of the nucleic acid sequences are expressed from different promoters on the expression vector. In some embodiments, each of the promoters can be the same or different from one another. For example, the at least one nucleic acid sequence encoding a transcriptional gene silencing element may be expressed from a first promoter; and each of the at least two other nucleic acid sequences are themselves expressed from separate promoters (e.g. second and third promoters). In other embodiments, two of the three nucleic acid sequences are transcribed from a single promoter (i.e. the first and second nucleic acid sequences or the second and third nucleic acid sequences). In yet other embodiments, three nucleic acid sequences are transcribed from a single promoter.

In further embodiments, each of the promoters (e.g. first, second, third, and optionally fourth promoters) can be RNA polymerase I (pol I), polymerase II (pol II), or polymerase III (pol III) promoters. The promoters may be constitutive promoters or inducible promoters as known to those of ordinary skill in the art. In some embodiments, the promoter contains at least a portion of an HIV LTR (e.g. TAR) and is inducible by HIV infection. In certain embodiments, the first promoter is a RNA pol III promoter. RNA pol III promoters suitable for use in the expression vectors of the disclosure include, but are not limited, to human U6, mouse U6, and human H1 others. In one embodiment, the first promoter is a H1 RNA pol III promoter. In other embodiments, the second promoter is a RNA pol II promoter. In one particular embodiment, the second promoter is a UbiquitinC pol II promoter. The second promoter, in some embodiments, can be a tissue-specific promoter. For instance, suitable tissue-specific promoters include macrophage-specific promoters (e.g., MPG-1 and the like) and T-cell promoters (e.g., CD4 and the like). In one embodiment, the third promoter is a RNA pol II promoter. In another embodiment, the third promoter is a UbiquitinC pol II promoter. The third promoter can, in some embodiments, be a tissue specific promoter. The first, second, and third promoters can be a combination of any of the promoters described herein. In certain embodiments, RNA pol III promoters are preferred where the nucleic acid sequence encodes an inhibitory RNA molecule, such as an siRNA or shRNA. In other embodiments, RNA pol II promoters are preferred where the nucleic acid sequence encodes a protein.

In some embodiments, the expression vector is a viral vector. In some embodiments, the viral vector is a lentiviral vector, as described herein. In other embodiments, the viral vector is a retroviral vector, also described herein.

"Retroviruses" are viruses having an RNA genome that is reverse transcribed by retroviral reverse transcriptase to a cDNA copy that is integrated into the host cell genome. Retroviral vectors and methods of making retroviral vectors are known in the art. Briefly, to construct a retroviral vector, a nucleic acid encoding a gene of interest is inserted into the viral genome in the place of certain viral sequences to produce a virus that is replication-defective. In order to produce virions, a packaging cell line containing the gag, pol, and env genes but without the LTR and packaging components is constructed (Mann et al., Cell, Vol. 33:153-159, 1983). When a recombinant plasmid containing a cDNA, together with the retroviral LTR and packaging sequences, is introduced into this cell line, the packaging sequence allows the RNA transcript of the recombinant plasmid to be packaged into viral particles, which are then secreted into the culture media. The media containing the recombinant retroviruses is then collected, optionally concentrated, and used for gene transfer.

"Lentivirus" refers to a genus of retroviruses that is capable of infecting dividing and non-dividing cells. Several examples of lentiviruses include HIV (human immunodeficiency virus: including HIV type 1, and HIV type 2), the etiologic agent of the human acquired immunodeficiency syndrome (AIDS); visna-maedi, which causes encephalitis (visna) or pneumonia (maedi) in sheep, the caprine arthritis-encephalitis virus, which causes immune deficiency, arthritis, and encephalopathy in goats; equine infectious anemia virus, which causes autoimmune hemolytic anemia, and encephalopathy in horses; feline immunodeficiency virus (FIV), which causes immune deficiency in cats; bovine immune deficiency virus (BIV), which causes lymphadenopathy, lymphocytosis, and possibly central nervous system infection in cattle; and simian immunodeficiency virus (SIV), which causes immune deficiency and encephalopathy in sub-human primates.

A lentiviral genome is generally organized into a 5' long terminal repeat (LTR), the gag gene, the pol gene, the env gene, the accessory genes (nef, vif, vpr, vpu) and a 3' LTR. The viral LTR is divided into three regions called U3, R and U5. The U3 region contains the enhancer and promoter elements. The U5 region contains the polyadenylation signals. The R (repeat) region separates the U3 and U5 regions and transcribed sequences of the R region appear at both the 5' and 3' ends of the viral RNA. See, for example, "RNA Viruses: A Practical Approach" (Alan J. Cann, Ed., Oxford University Press, (2000)); O Narayan and Clements (1989) J. Gen. Virology, Vol. 70:1617-1639; Fields et al. (1990) Fundamental Virology Raven Press.; Miyoshi H, Blamer U, Takahashi M, Gage F H, Verma I M. (1998) J Virol., Vol. 72(10):8150 7, and U.S. Pat. No. 6,013,516.

Lentiviral vectors are known in the art, including several that have been used to infect hematopoietic progenitor/stem cells (HPSC). Such vectors can be found, for example, in the following publications: Evans et al., Hum Gene Ther., Vol. 10:1479-1489, 1999; Case et al., Proc Natl Acad Sci USA, Vol. 96:2988-2993, 1999; Uchida et al., Proc Natl Acad Sci USA, Vol. 95:11939-11944, 1998; Miyoshi et al., Science, Vol. 283:682-686, 1999; and Sutton et al., J. Virol., Vol. 72:5781-5788, 1998. In one embodiment, the expression vector is a modified lentivirus, and thus is able to infect both dividing and non-dividing cells. Such lentiviral vectors comprise a modified lentiviral genome that comprises a first nucleic acid sequence encoding an inhibitor of an HIV co-receptor and a second nucleic acid sequence encoding a protein that inhibits HIV fusion to a target cell or HIV replication. Further, the modified lentiviral genome preferably lacks genes for lentiviral proteins required for viral replication, thus preventing undesired replication, such as replication in the target cells. The required proteins for replication of the modified genome are preferably provided in trans in the packaging cell line during production of the recombinant retrovirus (or specifically lentivirus). In one embodiment, the packaging cell line is a 293T cell line. The lentiviral vector preferably comprises sequences from the 5' and 3' long terminal repeats (LTRs) of a lentivirus. In one embodiment, the viral construct comprises the R and U5 sequences from the 5' LTR of a lentivirus and an inactivated or self-inactivating 3' LTR from a lentivirus. The LTR sequences may be LTR sequences from any lentivirus including from any species or strain. For example, the LTR may be LTR sequences from HIV, simian immunodeficiency virus (SIV), feline immunodeficiency virus (FIV) or bovine immunodeficiency virus (BIV). Preferably the LTR sequences are HIV LTR sequences.

### Transcriptional Gene Silencing Elements

The expression vectors of the present invention comprise one or more transcriptional gene silencing elements which target a sequence of the 5' LTR of HIV as recited in claim 1. In some embodiments, the expression vectors comprise at least two of the silencing nucleic acids which target a sequence of the 5' LTR of HIV. Without wishing to be bound by any particular theory, it is believed that the silencing nucleic acids that target a region within the 5' LTR sequence of HIV described herein are effective at inhibiting transcription of the HIV genome, and therefore represent a method by which HIV replication can be inhibited. Throughout this disclosure, the numbering of the silencing nucleic acids is based on the 5'LTR U3 start site in the subtype B HXB2 strain (Accession no. K03455) (Wong-Staal, F. 1985, Nature 313:277-284).

In some embodiments, the silencing nucleic acid targets a sequence having at least 95% identity with a target sequence from about position 143 to about position 161 of the 5' LTR of HIV-1. In some embodiments, the silencing nucleic acid targets a sequence having at least 95% identity with that of SEQ ID NO: 1. In other embodiments, the silencing nucleic acid targets a sequence having at least 97% identity with that of SEQ ID NO: 1. In further embodiments, the silencing nucleic acid targets a sequence having at least 99% identity with that of SEQ ID NO: 1. In yet further embodiments, the silencing nucleic acid targets a sequence having about 100% identity with that of SEQ ID NO: 1.

In some embodiments, the silencing nucleic acid targets a sequence having at least 95% identity with a target sequence from about position 136 to about position 154 of the 5' LTR of HIV-1. In some embodiments, the silencing nucleic acid targets a sequence having at least 95% identity with that of SEQ ID NO: 9. In yet other embodiments, the silencing nucleic acid targets a sequence having at least 97% identity with that of SEQ ID NO: 9. In further embodiments, the silencing nucleic acid targets a sequence having at least 99% identity with that of SEQ ID NO: 9. In yet further embodiments, the silencing nucleic acid targets a sequence having about 100% identity with that of SEQ ID NO: 9.

In some embodiments, the silencing nucleic acid targets a sequence having at least 95% identity with a target sequence from about position 205 to about position 223 of the 5' LTR of HIV-1. In some embodiments, the silencing nucleic acid targets a sequence having at least 95% identity with that of SEQ ID NO: 17. In other embodiments, the silencing nucleic acid targets a sequence having at least 97% identity with that of SEQ ID NO: 17. In further embodiments, the silencing nucleic acid targets a sequence having at least 99% identity with that of SEQ ID NO: 17. In yet further embodiments, the silencing nucleic acid targets a sequence having about 100% identity with that of SEQ ID NO: 17.

In some embodiments, the silencing nucleic acid targets a sequence having at least 95% identity with a target sequence from about position 350 to about position 368 of the 5' LTR of HIV-1. In some embodiments, the silencing nucleic acid targets a sequence having at least 95% identity with that of SEQ ID NO: 36. In other embodiments, the silencing nucleic acid targets a sequence having at least 97% identity with that of SEQ ID NO: 36. In further embodiments, the silencing nucleic acid targets a sequence having at least 99% identity with that of SEQ ID NO: 36. In yet further embodiments, the silencing nucleic acid targets a sequence having about 100% identity with that of SEQ ID NO: 36.

As will be appreciated by those skilled in the art, there may exist variation in the sequence of the 5' LTR region between strains or subtypes of HIV-1, and thus there may be some variation in the target sequence of HIV-1. In some embodiments, the silencing nucleic acid targets one or more sequences selected from SEQ ID NO: 2, SEQ ID NO: 3, SEQ ID NO: 4, or SEQ ID NO: 5, or a sequence having at least 95% identity to one of SEQ ID NO: 2, SEQ ID NO: 3, SEQ ID NO: 4, or SEQ ID NO: 5. In other embodiments, the silencing nucleic acid targets one or more sequences selected from SEQ ID NO: 10, SEQ ID NO: 11, SEQ ID NO: 12, or SEQ ID NO: 13, or a sequence having at least 95% identity to one of SEQ ID NO: 10, SEQ ID NO: 11, SEQ ID NO: 12, or SEQ ID NO: 13. In yet other embodiments, the silencing nucleic acid targets one or more sequences selected from SEQ ID NO: 18, SEQ ID NO: 19, SEQ ID NO: 20, SEQ ID NO: 21, or SEQ ID NO 33, or a sequence having at least 95% identity to one of SEQ ID NO: 18, SEQ ID NO: 19, SEQ ID NO: 20, SEQ ID NO: 21, or SEQ ID NO 33. In further embodiments, the silencing nucleic acid targets one or more sequences selected from SEQ ID NO: 37, SEQ ID NO: 38, or SEQ ID NO 39, or a sequence having at least 95% identity to one of SEQ ID NO: 37, SEQ ID NO: 38, or SEQ ID NO 39.

In some embodiments, the silencing nucleic acid which targets the sequence from about position 143 to about position 161 of the 5' LTR of HIV-1 is an RNA duplex comprising a sense strand and an antisense strand, wherein the sense strand comprises a sequence having at least 95% identity to that of SEQ ID NO: 6. In other embodiments, the silencing nucleic acid is an RNA duplex comprising a sense strand and an antisense strand, wherein the sense strand comprises a sequence having at least 97% identity to that of SEQ ID NO: 6. In further embodiments, the silencing nucleic acid is an RNA duplex comprising a sense strand and an antisense strand, wherein the sense strand comprises a sequence having at least 99% identity to that of SEQ ID NO: 6. In yet further embodiments, the silencing nucleic acid is an RNA duplex comprising a sense strand and an antisense strand, wherein the sense strand comprises a sequence having about 100% identity to that of SEQ ID NO: 6.

In some embodiments, the silencing nucleic acid which targets the sequence from about position 136 to about position 154 of the 5' LTR of HIV-1 is an RNA duplex comprising a sense strand and an antisense strand, wherein the sense strand comprises a sequence having at least 95% identity to that of SEQ ID NO: 14. In other embodiments, the silencing nucleic acid is an RNA duplex comprising a sense strand and an antisense strand, wherein the sense strand comprises sequence having at least 97% identity to that of SEQ ID NO: 14. In further embodiments, the silencing nucleic acid is an RNA duplex comprising a sense strand and an antisense strand, wherein the sense strand comprises a sequence having at least 99% identity to that of SEQ ID NO: 14. In yet further embodiments, the silencing nucleic acid is an RNA duplex comprising a sense strand and an antisense strand, wherein the sense strand comprises a sequence having about 100% identity to that of SEQ ID NO: 14.

In some embodiments, the silencing nucleic acid which targets the sequence from about position 205 to about position 223 of the 5' LTR of HIV-1 is an RNA duplex comprising a sense strand and an antisense strand, wherein the sense strand comprises a sequence having at least 95% identity to that of SEQ ID NO: 22. In other embodiments, the silencing nucleic acid is an RNA duplex comprising a sense strand and an antisense strand, wherein the sense strand comprises sequence having at least 97% identity to that of SEQ ID NO: 22. In further embodiments, the silencing nucleic acid is an RNA duplex comprising a sense strand and an antisense strand, wherein the sense strand comprises a sequence having at least 99% identity to that of SEQ ID NO: 22. In yet further embodiments, the silencing nucleic acid is an RNA duplex comprising a sense strand and an antisense strand, wherein the sense strand comprises a sequence having about 100% identity to that of SEQ ID NO: 22.

In some embodiments, the silencing nucleic acid which targets the sequence having at least 95% identity with a target sequence from about position 350 to about position 368 of the 5' LTR of HIV-1 is an RNA duplex comprising a sense strand and an antisense strand, wherein the sense strand comprises a sequence having at least 95% identity to that of SEQ ID NO: 40. In other embodiments, the silencing nucleic acid is an RNA duplex comprising a sense strand and an antisense strand, wherein the sense strand comprises sequence having at least 97% identity to that of SEQ ID NO: 40. In further embodiments, the silencing nucleic acid is an RNA duplex comprising a sense strand and an antisense strand, wherein the sense strand comprises a sequence having at least 99% identity to that of SEQ ID NO: 40. In yet further embodiments, the silencing nucleic acid is an RNA duplex comprising a sense strand and an antisense strand, wherein the sense strand comprises a sequence having about 100% identity to that of SEQ ID NO: 40.

The antisense strand of any RNA duplex comprises a sequence that is complementary to the sense strand. As used herein, a "sequence that is complementary to the sense strand" will be able to form a duplex despite having a less than 100% complementarity to the sense strand if at least a portion of the sequence is able to form a duplex with the sense strand. In some embodiments, the antisense strand is at least 95% complementary to the sense strand. In other embodiments, the antisense strand is at least 97% complementary to the sense strand. In further embodiments, the antisense strand is at least 99% complementary to the sense strand. In yet further embodiments, the antisense strand is about 100% complementary to the sense strand. It will be appreciated that the degree of identity between the sense and antisense strands of the RNA duplex may be different than the degree of identity between the sense strand and the respective target sequence.

The two strands forming the RNA duplex may be different portions of one larger RNA molecule, or they may be separate RNA molecules. Where the strands of the RNA duplex are formed from separate RNA molecules, the RNA duplex may be a "small interfering RNA" ("siRNA"). A "small interfering RNA" or "siRNA" is a double-stranded RNA molecule that is capable of inhibiting the expression of a gene with which it shares homology. The region of the gene or other nucleotide sequence over which there is homology is known as the "target region." Where the two strands are part of one larger molecule, and therefore are connected by a chain of nucleotides between the 3'-end of one strand and the 5' end of the other strand of the RNA duplex, the RNA duplex is a "short hairpin RNA" ("shRNA").

In some embodiments, the RNA duplex is a siRNA comprising a sense strand and an antisense strand. In one embodiment, the RNA duplex is a siRNA having a sense strand having the sequence of SEQ ID NO: 6, and an antisense strand having the sequence of SEQ ID NO: 7 (referred to herein as "si143" or "siRNA143"). In another embodiment, the RNA duplex is a siRNA having a sense strand having the sequence of SEQ ID NO: 14, and an antisense strand having the sequence of SEQ ID NO: 15 (referred to herein as "si136" or "siRNA136"). In a further embodiment, the RNA duplex is a siRNA having a sense strand having the sequence of SEQ ID NO: 22, and an antisense strand having the sequence of SEQ ID NO: 23 (referred to herein as "si205" or "siRNA205"). In yet a further embodiment, the RNA duplex is a siRNA having a sense strand having the sequence of SEQ ID NO: 40, and an antisense strand having the sequence of SEQ ID NO: 41 (referred to herein as "siRNA PromA" or "siPromA").

In other embodiments, the RNA duplex is a shRNA comprising a sense strand and an antisense strand. In one embodiment, the RNA duplex is a shRNA comprising a sense strand having the sequence of SEQ ID NO: 6 (or a sense strand having at least 95% identity to that of SED IQ NO: 6), and an antisense strand having the sequence of SEQ ID NO: 7. In yet another embodiment, the shRNA has the sequence of SEQ ID NO: 8 (referred to herein as "sh143" or "shRNA143"). In another embodiment, the RNA duplex is a shRNA comprising a sense strand having the sequence of SEQ ID NO: 14 (or a sense strand having at least 95% identity to that of SED IQ NO: 14), and an antisense strand having the sequence of SEQ ID NO: 15. In yet another embodiment, the shRNA has the sequence SEQ ID NO: 16 (referred to herein as "sh136" or "shRNA136"). In a further embodiment, the RNA duplex is a shRNA comprising a sense strand having the sequence of SEQ ID NO: 22 (or a sense strand having at least 95% identity to that of SED IQ NO: 22), and an antisense strand having the sequence of SEQ ID NO: 23. In yet another embodiment, the shRNA has the sequence of SEQ ID NO: 24 (referred to herein as "sh205" or "shRNA205"). In yet a further embodiment, the RNA duplex is a shRNA comprising a sense strand having the sequence of SEQ ID NO: 40 (or a sense strand having at least 95% identity to that of SED IQ NO: 40), and an antisense strand having the sequence of SEQ ID NO: 41. In yet another embodiment, the shRNA has the sequence of SEQ ID NO: 42 (referred to herein as "shPromA" or "shRNA PromA").

In other embodiments, the at least one silencing nucleic acid is a nucleic acid selected from the group consisting of antisense RNA, DNA or mixtures thereof; a DNAzyme; and a ribozyme; which target at least one of a sequence of SEQ ID NO: 1, a sequence of SEQ ID NO: 9, a sequence of SEQ ID NO: 17, or a sequence of SEQ ID NO: 36. Methods for the preparation and use of antisense RNA and DNA molecules, ribozymes and DNAzymes are known in the art and are described in, for example, Jakobsen et al, 2007, Retrovirology 4: 29-41.

In other embodiments, the silencing nucleic acid may be a DNA polynucleotide which encodes the sense and/or antisense sequence of the siRNA or shRNA. Such DNA sequences may be inserted into vectors, such as plasmids, viral vectors, etc., to achieve expression of the siRNA or shRNA in the cell. In the case of siRNA, the sense and antisense strands of the siRNA may be expressed from the same or different vectors.

### Nucleic Acid Sequences Which Encode Inhibitors of an HIV co-receptor

In some embodiments, the inhibitor of an HIV co-receptor is an inhibitory nucleic acid, including a siRNA, a shRNA, an aptamer, a ribozyme, and an antisense oligonucleotide. Thus, in some embodiments, a nucleic acid sequence of an expression vector encodes an inhibitory nucleic acid that targets an HIV co-receptor. "Target" refers to the ability of the inhibitor to bind to and/or interfere with an endogenous transcript encoding the HIV co-receptor. For instance, the inhibitory nucleic acid can have a sequence that is substantially complementary to a nucleic acid encoding the HIV co-receptor such that the inhibitory nucleic acid binds to the HIV co-receptor-encoding nucleic acid thereby blocking the expression or initiating the degradation of the co-receptor nucleic acid. Accordingly, in some embodiments, the inhibitor of an HIV co-receptor is capable of reducing expression of the HIV co-receptor when the expression vector encoding the inhibitor is expressed in a host cell.

In some embodiments, an expression vector of the present disclosure comprises a nucleic acid sequence encoding an antisense oligonucleotide having a sequence that is substantially complementary to at least a portion of a nucleic acid sequence encoding an HIV co-receptor. Examples of HIV co-receptors include CCR5 and/or CXCR4. As used herein, "substantially complementary" refers to a sequence that is at least about 75%, 80%, 85%, 90%, 95%, 96%, 97%, 98%, or 99% complementary to a target polynucleotide sequence. In one embodiment, the antisense oligonucleotide has a sequence that is 100% complementary to at least a portion of a nucleic acid sequence encoding CCR5 or CXCR4. In some embodiments, the antisense oligonucleotide can be from about 15 to about 30 nucleotides in length. In other embodiments, the antisense oligonucleotide can be from about 19 to about 25 nucleotides in length.

In certain embodiments, a nucleic acid sequence of the expression vector encodes a shRNA having a stem-loop structure, wherein the stem or double-stranded region comprises a first portion of the double-stranded region having a sequence that is identical to part of the sequence of the HIV co-receptor, and wherein a second portion of the double-stranded region comprises a sequence that is complementary to another part of the sequence of the HIV co-receptor. In some embodiments, the loop region of the shRNA can comprise from about 2 to about 19 nucleotides. In one particular embodiment, the first nucleic acid sequence encodes a shRNA comprising the sequence of SEQ ID NO: 25. In another particular embodiment, the shRNA loop structure comprises the sequence of SEQ ID NO:43.

### Nucleic Acid Sequences Which Encode a Protein That Inhibits HIV Fusion

In some embodiments, the expression vectors of the present disclosure comprise a nucleic acid sequence encoding a protein that inhibits HIV fusion to a target cell. In some embodiments, the protein that inhibits HIV fusion to a target cell is a C46 protein. C46 is a membrane anchored fusion inhibitor derived from the C-terminal heptad repeat of HIV gp41 fused with a human immunoglobulin hinge region and a CD34 transmembrane domain. C46 is a potent HIV fusion inhibitor, which the skilled artisan will appreciate that, in a sense, is analogous to the FDA approved soluble drug enfuvirtide (T20). While analogous, the C46 protein is not T20 (e.g. the C46 peptide is about 10 amino acids longer in length than T20) (see, for example, Petit et al. Human Gene Therapy Methods. August 2014, 25(4): 232-240. doi:10.1089/hgtb.2014.034, and Felix et al. Mutations in gp120 Contribute to the Resistance of Human Immunodeficiency Virus Type 1 to Membrane-Anchored C-Peptide maC46, J Virol. 2009 May; 83(10): 4844-4853). The skilled artisan will also appreciate that the nucleic acid sequence which encodes a protein that inhibits HIV fusion (e.g. C46 or T20) acts at a point in the HIV life cycle distinct from CCR5 or CXCR4 co-receptor attachment. In some embodiments, the safety of C46 was tested in a phase I clinical trial in which patients received an infusion of autologous T-cells transduced with C46 retroviral vector. Without wishing to be bound by any particular theory, that study indicated that the patients had no gene therapy related adverse effects and did not develop apparent anti-C46 immune reactions.

In some embodiments, the expression vectors of the present disclosure comprise a nucleic acid sequence encoding a protein that inhibits HIV fusion to a target cell other than T20, i.e. the nucleic acid encoding an HIV fusion inhibitor is not T20.

In one embodiment, a nucleic acid sequence of an expression vector encodes a C46 protein comprising the sequence of SEQ ID NO: 26.

In another embodiment, a nucleic acid sequence of an expression vector comprises the sequence of SEQ ID NO: 27.

The skilled artisan will appreciate that other suitable proteins that inhibit HIV fusion to a target cell and can be encoded by a nucleic acid sequence in the expression vectors of the present disclosure and include, without limitation, T20 and its related proteins, enfuvirtide, CP32M, and sifuvirtide (see, e.g., PCT/US2010/036247, published as WO 2011/008348 A2).

### Nucleic Acid Sequences Which Encode a Protein That Inhibits HIV Replication

In some embodiments, the expression vectors of the present disclosure comprise a nucleic acid sequence encoding a protein that inhibits HIV replication. In some embodiments, the nucleic acid sequence encodes a tripartite motif-containing 5 alpha (TRIM5α) protein or a derivative or fusion thereof. In other embodiments, the nucleic acid sequence encodes a chimeric TRIM5α. A "chimeric TRIM5α" refers to a TRIM5α protein comprising domains or fragments from TRIM5α proteins from two or more species. In some embodiments, the chimeric TRIM5α comprises an amino terminal domain from a human TRIM5α protein and the carboxy terminal PRYSPRY domain is from a rhesus TRIM5α protein.

In some embodiments, the nucleic acid sequence encodes a TRIM5-cyclophilin fusion protein. In some embodiments, the TRIM5-cyclophilin fusion protein comprises amino acids 1 to about 309 of human TRIM5α fused directly to about full-length human cyclophilin A. In other embodiments, the TRIM5-cyclophilin fusion protein comprises amino acids 1 to about 322 of human TRIM5α fused directly to about full-length human cyclophilin A. In yet other embodiments, the TRIM5-cyclophilin fusion protein comprises amino acids 1 to about 331 of human TRIM5α fused directly to about full-length human cyclophilin A. Further suitable proteins that inhibit HIV replication that can be encoded by the second nucleic acid sequence include, but are not limited to, cyclophilin, E3 ubiquitin, APOBEC3G, and bone marrow stromal cell antigen 2 (BST-2). In some embodiments, the expression vector comprises a nucleic acid sequence which encodes a human TRIM5α protein as provided by SEQ ID NO:28. In other embodiments, the expression vector comprises a nucleic acid sequence having the sequence of SEQ ID NO:29.

### Examples of Expression Vectors

In some embodiments, the present disclosure provides an expression vector comprising a first nucleic acid sequence encoding an inhibitor of an HIV co-receptor, a second nucleic acid sequence encoding a protein that inhibits HIV fusion to the target cell, and a third nucleic acid encodes a silencing nucleic acid which targets a sequence of the 5' LTR of HIV-1 as recited in claim 1, to inhibit HIV-1 gene transcription, wherein the first nucleic acid sequence is operably linked to a first promoter, the second nucleic acid sequence is operably linked to a second promoter, and the third nucleic acid is operably linked to a third promoter. In some embodiments, the first and second nucleic acid sequences are transcribed from a single promoter. In other embodiments, two of the three nucleic acid sequences are transcribed from a single promoter. In some embodiments, the second nucleic acid sequence encoding a protein that inhibits HIV fusion to the target cell is selected from a protein other than T20, i.e. the protein is not T20. In some embodiments, the protein other than T20 is C46.

In other embodiments, the present disclosure provides an expression vector comprising a first nucleic acid sequence which encodes an siRNA or shRNA molecule which comprises a double-stranded region having a sequence that is substantially identical and complementary to CCR5; a second nucleic acid sequence which encodes a C46 protein; and a third nucleic acid sequence which encodes a silencing nucleic acid which targets a sequence of the 5' LTR of HIV to inhibit HIV gene transcription as recited in claim 1; wherein the first nucleic acid sequence is operably linked to a first promoter, the second nucleic acid sequence is operably linked to a second promoter, and the third nucleic acid is operably linked to a third promoter. In some embodiments, the first and second nucleic acid sequences are transcribed from a single promoter. In other embodiments, two of the three nucleic acid sequences are transcribed from a single promoter. In some embodiments, the expression vector comprises a fourth nucleic acid sequence, such as one encoding another transcriptional gene silencing element.

In yet other embodiments, the present disclosure provides an expression vector which comprises a first, second, third and fourth nucleic acid sequence, wherein the first nucleic acid sequence encodes an inhibitor of an HIV co-receptor (e.g., shRNA to CCR5 or CXCR4), the second nucleic acid sequence encodes a fusion inhibitor (e.g., C46), the third nucleic acid sequence encodes an inhibitor of HIV replication (e.g., TRIM5α protein or a derivative or fusion thereof), and the fourth nucleic acid sequence encodes a silencing nucleic acid which targets one of a sequence from about position 143 to about position 161 of the 5' LTR of HIV-1, a sequence from about position 136 to about position 154 of the 5' LTR of HIV-1, a sequence from about position 205 to about position 223 of the 5' LTR of HIV-1, or a sequence from about position 350 to about position 368 of the 5' LTR of HIV-1 to inhibit HIV-1 gene transcription. In some embodiments of the disclosure, the inhibitor of HIV-1 gene transcription, the inhibitor of an HIV co-receptor and the inhibitor of HIV fusion to the target cell or inhibitor of HIV replication are expressed from different promoters on the expression vector. In one embodiment, the inhibitor of an HIV co-receptor (e.g. CCR5 or CXCR4) is expressed from a RNA polymerase III promoter, while the inhibitor of HIV fusion and/or replication is expressed from a RNA polymerase II promoter. The person of ordinary skill in the art will appreciate that two different inhibitors can be expressed in different ratios from the expression construct.

Other examples of expression vectors of the present disclosure are illustrated in Figure 4. Figure 4 also illustrates specific promoters to which each nucleic acid of the expression vector is operably linked.

### Compositions and Pharmaceutical Compositions

The present disclosure also provides for compositions, including pharmaceutical compositions, comprising one or more expression vectors as disclosed herein. By way of example, a composition may comprise an expression vector comprising a first nucleic acid comprising a transcriptional gene silencing element which targets a sequence within the 5' LTR of HIV, a second nucleic acid encoding a protein that inhibits HIV fusion to a target cell or HIV replication, and a third nucleic acid encoding an inhibitor of an HIV co-receptor. Another example is a composition comprises an expression vector having a first nucleic acid sequence encoding a shRNA targeting CCR5 (or CXCR4), a second nucleic acid sequence encoding a C46 protein, and a third nucleic acid, namely a silencing nucleic acid, which targets a sequence of the 5' LTR of HIV.

In some examples, the present disclosure also provides for compositions comprising (i) an expression vector or a composition as disclosed in U.S. Publication No. 2012/0201794; and (ii) at least one transcriptional gene silencing element which targets a sequence within the 5' LTR of HIV, as disclosed herein. For example, in some examples, such a composition may comprise (i) an expression vector comprising at least two nucleic acid sequences selected from the group consisting of a nucleic acid sequence that encodes an inhibitor of an HIV co-receptor; a nucleic acid sequence that encodes a protein that inhibits HIV fusion to a target cell; a nucleic acid sequence that encodes a protein that inhibits HIV replication; and a nucleic acid sequence that encodes an inhibitor of viral entry; and (ii) at least one transcriptional gene silencing element or an expression vector, cell, or composition comprising at least one transcriptional gene silencing element. By way of example, such a composition may comprise (i) an expression vector having a first nucleic acid sequence encoding a shRNA targeting CCR5 (or CXCR4), and a second nucleic acid sequence encoding a C46 protein; and (ii) a silencing nucleic acid which targets a sequence of the 5' LTR of HIV.

In some examples, pharmaceutical compositions comprise an effective amount of at least one of the expression vectors or compositions as described herein and a pharmaceutically acceptable carrier. For instance, in certain examples, the pharmaceutical composition comprises an effective amount of an expression vector and a pharmaceutically acceptable carrier. As used herein, an "effective amount" is an amount sufficient to cause inhibition of HIV gene expression. An affective amount can be readily determined by those skilled in the art based on factors such as body size, body weight, age, health, sex of the subject, ethnicity, and viral titers.

Any of the expression vectors disclosed herein (or combinations thereof) may be formulated as a pharmaceutical composition. The phrases "pharmaceutically acceptable" or "pharmacologically acceptable" refer to molecular entities and compositions that do not produce adverse, allergic, or other untoward reactions when administered to an animal or a human. For example, the silencing nucleic acid may be formulated with a pharmaceutically acceptable carrier. As used herein, "pharmaceutically acceptable carrier" includes solvents, buffers, solutions, dispersion media, coatings, antibacterial and antifungal agents, isotonic and absorption delaying agents and the like acceptable for use in formulating pharmaceuticals, such as pharmaceuticals suitable for administration to humans. Methods for the formulation of compounds with pharmaceutical carriers are known in the art and are described in, for example, in Remington's Pharmaceutical Science, (17th ed. Mack Publishing Company, Easton, Pa. 1985); and Goodman & Gillman's: The Pharmacological Basis of Therapeutics (11th Edition, McGraw-Hill Professional, 2005).

The pharmaceutical compositions may comprise any of the expression vectors or compositions disclosed herein (or combinations thereof) in any concentration that allows the silencing nucleic acid administered to achieve a concentration in the range of from about 0.1 mg/kg to about 1 mg/kg. The pharmaceutical compositions may comprise the expression vector (or combinations thereof) in an amount of from about 0.1% to about 99.9% by weight. Pharmaceutically acceptable carriers suitable for inclusion within any pharmaceutical composition include water, buffered water, saline solutions such as, for example, normal saline or balanced saline solutions such as Hank's or Earle's balanced solutions), glycine, hyaluronic acid etc. The pharmaceutical composition may be formulated for parenteral administration, such as intravenous, intramuscular or subcutaneous administration. Pharmaceutical compositions for parenteral administration may comprise pharmaceutically acceptable sterile aqueous or non-aqueous solutions, dispersions, suspensions or emulsions as well as sterile powders for reconstitution into sterile injectable solutions or dispersions. Examples of suitable aqueous and non-aqueous carriers, solvents, diluents or vehicles include water, ethanol, polyols (such as glycerol, propylene glycol, polyethylene glycol, etc.), carboxymethylcellulose and mixtures thereof, vegetable oils (such as olive oil), injectable organic esters (e.g. ethyl oleate).

The pharmaceutical compositions may comprise any of the expression vectors disclosed herein (or combinations thereof) in an encapsulated form. For example, the expression vectors may be encapsulated by biodegradable polymers such as polylactide-polyglycolide, poly(orthoesters) and poly(anhydrides), or may be encapsulated in liposomes or microemulsion. Liposomes may be, for example, lipofectin or lipofectamine. Another example may comprise the expression vectors disclosed herein (or combinations thereof) in or on anucleated bacterial minicells (Giacalone et al, Cell Microbiology 2006, 8(10): 1624-33). The expression vectors disclosed herein (or combinations thereof) may be combined with nanoparticles.

The pharmaceutical composition may be formulated for oral administration. Solid dosage forms for oral administration may include, for example, tablets, dragees, capsules, pills, and granules. In such solid dosage forms, the composition may comprise at least one pharmaceutically acceptable carrier such as sodium citrate and/or dicalcium phosphate and/or fillers or extenders such as starches, lactose, sucrose, glucose, mannitol, and silicic acid; binders such as carboxylmethylcellulose, alginates, gelatin, polyvinylpyrrolidone, sucrose and acacia; humectants such as glycerol; disintegrating agents such as agar-agar, calcium carbonate, potato or tapioca starch, alginic acid, silicates, and sodium carbonate; wetting agents such as acetyl alcohol, glycerol monostearate; absorbants such as kaolin and bentonite clay; and/or lubricants such as talc, calcium stearate, magnesium stearate, solid polyethylene glycol, sodium lauryl sulfate, and mixtures thereof. Liquid dosage forms for oral administration may include, for example, pharmaceutically acceptable emulsions, solutions, suspensions, syrups and elixirs. Liquid dosages may include inert diluents such as water or other solvents, solubilizing agents and/or emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (such as, for example, cottonseed oil, corn oil, germ oil, castor oil, olive oil, sesame oil), glycerol, tetrahydrofurfuryl alcohol, polyethylene glycols and fatty acid esters of sorbitan, and mixtures thereof.

The pharmaceutical compositions may comprise penetration enhancers to enhance their delivery. Penetration enhancers may include fatty acids such as oleic acid, lauric acid, capric acid, myristic acid, palmitic acid, stearic acid, linoleic acid, linolenic acid, dicaprate, reclineate, monoolein, dilaurin, caprylic acid, arachidonic acid, glyceryl 1-monocaprate, mono and di-glycerides and physiologically acceptable salts thereof. The compositions may further include chelating agents such as, for example, ethylenediaminetetraacetic acid (EDTA), citric acid, salicylates (e.g. sodium salycilate, 5-methoxysalicylate, homovanilate). The expression vectors disclosed herein (or combinations thereof) may be delivered combined with minicells or nanoparticles.

In some embodiments, the compositions may be formulated as nanocapsules. Nanocapsule structure consists of nano-vesicular system that is formed in a core-shell arrangement. The shell of a typical nanocapsule is made of a polymeric membrane or coating. In some embodiments, the nanocapsules are derived from a biodegradable or bioerodable polymeric material.

In some embodiments, the nanocapsule is an enzymatically degradable nanocapsule. In some embodiments, the nanocapsule consists of a single-protein core and a thin polymeric shell cross-linked by peptides. In some embodiments, a nanocapsule may be selected such that it is specifically recognizable and able to be cleaved by a protease. In some embodiments, the cleavable cross-linkers include a peptide sequence or structure that is a substrate of a protease or another enzyme. Suitable nanocapsules, methods of synthesis, and/or methods of encapsulation, are further disclosed in United States Patent Publication No. 2011/0274682.

In other embodiments, the compositions may be formulated as bio-nanocapsules, which are nano-size capsules produced by a genetically engineered microorganism. It is possible to use, as a bio-nanocapsule, a virus protein-derived or modified virus protein-derived particle, such as a virus surface antigen particle (e.g., a hepatitis B virus surface antigen (HBsAg) particle). It is also possible to use, as a bio-nanocapsule, a nano-size capsule comprising a lipid bilayer membrane and a virus protein-derived or modified virus protein-derived particle such as a virus surface antigen particle (e.g., a hepatitis B virus surface antigen (HBsAg) particle). Such particles can be purified from eukaryotic cells such as yeasts, insect cells, and mammalian cells. The size of a capsule that can be used is approximately 10 nm to 500 nm, preferably 20 nm to 250 nm, and most preferably 80 nm to 150 nm can be used.

### Medical Uses

In general, the present disclosure provides expression vectors for various medical uses, including expression vectors for use in (i) methods of inhibiting HIV transcription; (ii) methods of inhibiting HIV replication; (iii) methods of treating an HIV infection; (iv) methods of preventing an HIV infection; (v) methods of reducing an HIV infection; (vi) methods of preventing or reducing a productive HIV infection in a subject not suffering from any HIV infection; (vii) methods of inhibiting binding an HIV co-receptor; and (viii) methods of inhibiting fusion of HIV to a target cell. These methods are to be carried out or achieved by administering, or contacting a cell with, an expression vector as described herein, a composition comprising an expression vector and/or other component or active agents, or co-administering (a) composition comprising an expression vector, and (b) another component or active agent. The present disclosure also provides the present expression vectors, compositions comprising expression vectors, etc. for use in a method to inhibit HIV gene transcription, binding to HIV co-receptor, fusion of HIV to the target cell or HIV replication for treating or preventing HIV infection in a subject.

More specifically, the present disclosure provides an expression vector for use in a method of treating, preventing, and/or reducing HIV infection in a patient or subject, such as those in need to treatment or therapy. As used herein, "treating" means affecting a subject, tissue or cell to obtain a desired pharmacological and/or physiological effect and includes inhibiting the condition, i.e. arresting its development; or relieving, mitigating or ameliorating the effects of the condition i.e. cause reversal or regression of the effects of the condition. "Reducing" means to lower the degree or intensity of a condition from occurring in a cell or a subject that may be at risk of having the condition. As used herein, "preventing" means preventing a condition from occurring in a cell or subject that may be at risk of having the condition, but does not necessarily mean that condition will not eventually develop, or that a subject will not eventually develop a condition. Preventing includes delaying the onset of a condition in a cell or subject. In one embodiment, treating achieves the result of preventing or reducing HIV replication. Treating may also achieve the result of preventing reactivation of latent HIV1- virus in the recipient subject.

In some embodiments, an expression vector for use in preventing and/or reducing HIV infection in a subject comprises an expression vector for use in preventing and/or reducing HIV infection in a subject suffering from HIV infection. As used herein, the expression "preventing or reducing HIV infection in a subject suffering from HIV infection" refers to eliminating, reducing or delaying the production of infectious HIV virus in a subject already infected with HIV such that infection of uninfected tissue with HIV in the subject is prevented, reduced or delayed. In another embodiment, an expression vector for use in preventing or reducing HIV infection in a subject comprises an expression vector for use in preventing or reducing a productive HIV infection in a subject not suffering from HIV infection. As used herein, " preventing or reducing a productive HIV infection in a subject not suffering from HIV infection" refers to an expression vector for use in preventing or reducing development of an HIV infection of a subject who has not been previously infected with HIV.

As used herein, the term "subject" refers to a mammal that is susceptible to HIV infection, such as a human, mouse or primate. Typically, the mammal is a human (homo sapiens).

As used herein, the term "administering" means providing a composition, formulation, or specific agent to a subject in need of treatment, including those described herein.

It will be understood that the specific dose level and frequency of dosage for any particular subject may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

In some embodiments, the methods disclosed herein are to comprise administering to the subject an effective amount of an expression vector or pharmaceutical composition comprising an expression vector as provided herein. Administration of such compositions can confer resistance to infection by R5 and X4 tropic strains of HIV. In some embodiments, the subject is human. The subject may be HIV negative or HIV positive. In some embodiments, the subject may be naive to cART therapy, receiving cART therapy, failing or failed on cART therapy. In other embodiments, the subject may have progressed to AIDS or have an AIDS-defining illness (e.g., AIDS/lymphoma).

By way of example, in some embodiments, the methods are to comprise administering to the subject a pharmaceutical composition comprising an expression vector, wherein the expression vector comprises a first nucleic acid sequence encoding a shRNA targeting CCR5 (or CXCR4), a second nucleic acid sequence encoding a C46 protein, and a third nucleic acid sequence encoding a silencing nucleic acid which targets a sequence in the 5' LTR of HIV as recited in claim 1.

In some embodiments the expression vector is for use in a method of treating an HIV infection in a subject comprising administering to the subject an effective amount of an expression vector or a composition comprising an expression vector, wherein the expression vector comprises (i) at least one nucleic acid sequence encoding a transcriptional gene silencing element as recited in claim 1; and (ii) at least two other nucleic acid sequences selected from the group consisting of a nucleic acid sequence that encodes a nucleic acid molecule that inhibits an HIV co-receptor; a nucleic acid sequence that encodes an HIV fusion inhibitor protein; a nucleic acid sequence encoding an inhibitor of HIV replication; and a nucleic acid sequence encoding an inhibitor of viral entry. In other embodiments, the expression vector is for use in a method of preventing and/or reducing an HIV infection in a subject comprising administering to the subject an effective amount of an expression vector or a composition comprising an expression vector, wherein the expression vector comprises (i) at least one nucleic acid sequence encoding a transcriptional gene silencing element as recited in claim 1; and (ii) at least two other nucleic acid sequences selected from the group consisting of a nucleic acid sequence that encodes a nucleic acid molecule that inhibits an HIV co-receptor; a nucleic acid sequence that encodes an HIV fusion inhibitor protein; a nucleic acid sequence encoding an inhibitor of HIV replication; and a nucleic acid sequence encoding an inhibitor of viral entry.

In other embodiments the expression vector is for use in a method of treating, preventing, and/or reducing an HIV infection in a subject comprising administering to the subject an effective amount of an expression vector or a composition comprising an expression vector, wherein the expression vector comprises a first nucleic acid sequence encoding an inhibitory nucleic acid capable of reducing expression of an HIV co-receptor; a second nucleic acid sequence encoding an HIV fusion inhibitor protein; and a third nucleic acid sequence encoding a silencing nucleic acid that targets a sequence of a 5' LTR of HIV, wherein the third nucleic acid is selected from the group consisting of (i) a silencing nucleic acid which targets a sequence from about position 143 to about position 161 of the 5' LTR of HIV-1, (ii) a silencing nucleic acid which targets a sequence from about position 136 to about position 154 of the 5' LTR of HIV-1, (iii) a silencing nucleic acid which targets a sequence from about position 205 to about position 223 of the 5' LTR of HIV-1; and (iv) a silencing nucleic acid which targets a sequence from about position 350 to about position 368 of the 5' LTR of HIV-1. In some embodiments, the nucleic acid encoding an HIV fusion inhibitor is other than T20.

In other examples the expression vector is for use in a method of treating, preventing, and/or reducing HIV infection in a subject, comprising administering to the subject an effective amount of a composition comprising (i) an expression vector comprising at least two nucleic acids, wherein the at least two nucleic acids are selected from the group consisting of a nucleic acid sequence that encodes a nucleic acid molecule that inhibits an HIV co-receptor; a nucleic acid sequence that encodes an HIV fusion inhibitor protein; a nucleic acid sequence encoding an inhibitor of HIV replication; and a nucleic acid sequence encoding an inhibitor of viral entry; and (ii) a nucleic acid that encodes at least one transcriptional gene silencing element which targets a sequence of the 5' LTR of HIV.

In yet other examples, the expression vector is for use in methods of treating an HIV infection in a subject comprising co-administering (i) an effective amount of an expression vector, a pharmaceutical composition comprising an expression vector, or a cell comprising at least two nucleic acids, wherein the at least two nucleic acids are selected from the group consisting of a nucleic acid sequence that encodes an inhibitor of an HIV co-receptor; a nucleic acid sequence that encodes a protein that inhibits HIV fusion to a target cell; a nucleic acid sequence that encodes a protein that inhibits HIV replication; and a nucleic acid sequence that encodes an inhibitor of viral entry; and (ii) an effective amount of at least one silencing nucleic acid which targets a sequence in the 5' LTR of HIV. In some examples, the co-administering is simultaneous. In other examples, the expression vector and the at least one transcriptional gene silencing element are administered at different times.

In yet further examples, the expression vector is for use in methods of treating, preventing, and/or reducing HIV infection in a subject comprising co-administering (i) an effective amount of an expression vector, a pharmaceutical composition comprising an expression vector, or a cell as disclosed in U.S. Publication No. 2012/0201794 (wherein the expression vectors from the '794 Publication are referred to as "Cal-1" herein); and (ii) an effective amount of at least one transcriptional gene silencing element, a composition or conjugate comprising at least one transcriptional gene silencing element, or a cell comprising at least one transcriptional gene silencing element. In some examples, the transcriptional gene silencing element is selected from those described in PCT/AU2015/050507, filed August 28, 2015, which is published as WO 2016/029275 A1. In some examples, the co-administering is simultaneous. In other examples, the expression vector and the at least one transcriptional gene silencing element are administered at different times. The skilled artisan will be able to provide an appropriate dosing regime and/or schedule for coadministration of the expression vector and the transcriptional gene silencing element.

Yet further embodiments relate to an in vitro method for treating human hematopoietic stem cells infected with HIV comprising providing hematopoietic stem cells extracted from circulating blood or bone marrow of a host patient; and treating the hematopoietic cells with the expression vector of the invention, wherein the transduced hematopoietic cells are to be transplanted in the subject, wherein the transduced hematopoietic cells are resistant to HIV infection, wherein the expression vector comprises a first nucleic acid sequence encoding an inhibitory nucleic acid capable of reducing expression of an HIV co-receptor; a second nucleic acid sequence encoding an HIV fusion inhibitor protein; and a third nucleic acid sequence encoding a silencing nucleic acid that targets a sequence of a 5' LTR of HIV selected from the group consisting of (i) a silencing nucleic acid which targets a sequence from about position 143 to about position 161 of the 5' LTR of HIV-1, (ii) a silencing nucleic acid which targets a sequence from about position 136 to about position 154 of the 5' LTR of HIV-1, (iii) a silencing nucleic acid which targets a sequence from about position 205 to about position 223 of the 5' LTR of HIV-1; and (iv) a silencing nucleic acid which targets a sequence from about position 350 to about position 368 of the 5' LTR of HIV-1. The transducing hematopoietic cells are, e.g., HPSC, CD4+ T lymphocytes, CD8+ T lymphocytes, or monocyte/macrophages. In some embodiments, the hematopoietic cells are hematopoietic progenitor/stem cells (HPSC) that generate granulocytes, monocyte/macrophages, and lymphocytes that are resistant to HIV infection following transplantation into a patient. In some embodiments, the HPSC are autologous and CD34 positive. In some embodiments, the transduced HPSC can generate granulocytes, monocyte/macrophages, and lymphocytes that are resistant to infection by R5 and X4 tropic strains of HIV. In some embodiments, the transduced HPSC can generate granulocytes, monocyte/macrophages, and lymphocytes that are resistant to infection by HIV strains that are resistant to cART.

In other embodiments, the invention provides an in vitro method for treating human hematopoietic stem cells infected with HIV, the method comprising providing hematopoietic stem cells extracted from the circulating blood or bone marrow of a human host patient; and treating the hematopoietic stem cells with an expression vector (or a composition comprising an expression) as disclosed herein; wherein the and re- treated hematopoietic stem cells are to be administered or transplanted into the same patient and wherein, the expression vector comprises a first nucleic acid sequence encoding an inhibitory nucleic acid capable of reducing expression of an HIV co-receptor; a second nucleic acid sequence encoding an HIV fusion inhibitor protein; and a third nucleic acid sequence encoding a silencing nucleic acid that targets a sequence of a 5' LTR of HIV selected from the group consisting of (i) a silencing nucleic acid which targets a sequence from about position 143 to about position 161 of the 5' LTR of HIV-1, (ii) a silencing nucleic acid which targets a sequence from about position 136 to about position 154 of the 5' LTR of HIV-1, (iii) a silencing nucleic acid which targets a sequence from about position 205 to about position 223 of the 5' LTR of HIV-1; and (iv) a silencing nucleic acid which targets a sequence from about position 350 to about position 368 of the 5' LTR of HIV-1.

### Host Cells and Methods of Making Host Cells

The present invention also provides a host cell comprising the novel expression vectors of the present invention. A "host cell" or "target cell" means a cell that is to be transformed using the methods and expression vectors of the disclosure. In some embodiments, the host cells are mammalian cells in which the expression vector can be expressed. Suitable mammalian host cells include, but are not limited to, human cells, murine cells, non-human primate cells (e.g. rhesus monkey cells), human progenitor cells or stem cells, 293 cells, HeLa cells, D17 cells, MDCK cells, BHK cells, and Cf2Th cells. In certain embodiments, the host cell comprising an expression vector of the invention is a hematopoietic cell, such as hematopoietic progenitor/stem cell (e.g. CD34-positive hematopoietic progenitor/stem cell (HPSC)), a monocyte, a macrophage, a peripheral blood mononuclear cell, a CD4+ T lymphocyte, a CD8+ T lymphocyte, or a dendritic cell. In some embodiments, the host cell is a CCR5+ hematopoietic cell. In other embodiments, the host cell may be a host cell from a patient or matched to a patient. In certain embodiments, a host cell transduced with the expression vectors of the disclosure are resistant to infection by X4 or R5-tropic HIV strains, including HAART resistant strains.

The hematopoietic cells (e.g. HPSC, CD4+ T lymphocytes, CD8+ T lymphocytes, and/or monocyte/macrophages) to be transduced with an expression vector of the invention can be allogeneic or autologous. The HPSC are, in some embodiments, CD34-positive and can be isolated from the patient's bone marrow or peripheral blood. The isolated CD34-positive HPSC (and/or other hematopoietic cell described herein) is, in some embodiments, transduced with an expression vector of the invention as described herein. For example, in one embodiment the expression vector comprises a first nucleic acid sequence encoding an inhibitor of an HIV co-receptor, a second nucleic acid sequence encoding a protein that inhibits HIV fusion to a target cell, and a third nucleic acid sequence that encodes a transcriptional gene silencing element which targets a sequence within the 5' LTR of HIV as recited in claim 1.

Following transduction of the hematopoietic cells (e.g., FIPSC, CD4+ T lymphocytes, CD8+ lymphocytes, or monocyte/macrophages) with an expression vector as disclosed herein, the transduced cells are for reintroduction or transplantation back into the patient. The transduced cells can be for injection parenterally into the patient, or for reintroduction by any other route known in the art. In some embodiments, the transduced hematopoietic cells are for injection intravenously into the patient. In some embodiments, the hematopoietic cell is a HPSC and an effective dose is an amount that is sufficient to at least partially reconstitute the immune system with HIV-resistant cells. The dose could be for administration in one or more administrations and may be from about 0.5 × 106 HPSC per kg patient weight to about 1×109 HPSC per kg patient weight. In another embodiment, the hematopoietic cell is a CD4+ T lymphocyte, a CD8+ T lymphocyte, or a monocyte/macrophage and an effective dose may be from about 1×109 cells per patient to 1×1011 cells per patient. However, the precise determination of what would be considered an effective dose may be based on factors individual to each patient, including their size, age, severity of HIV infection (e.g. viral titer), and amount of time since contraction of the virus. One skilled in the art, specifically a physician, would be able to determine the number of transduced hematopoietic cells which would constitute an effective dose without being subjected to undue experimentation.

### Kits

In some aspects the present disclosure provides a kit comprising an expression vector or a composition comprising an expression as described herein. The kit may comprise a container, where the container may be a bottle comprising the expression vector or composition in oral or parenteral dosage form, each dosage form comprising a unit dose of the expression vector or composition comprising the expression vector. The kit may comprise a label or the like, indicating treatment of a subject according to the present method.

### Detailed Methodology

### Synthesis

The present disclosure also includes a method of producing a viral expression vector that is capable of inhibiting binding of HIV to the cell and preventing HIV fusion to the cell and/or HIV replication when expressed in a host cell. In one example, the method comprises synthesizing a cDNA of a gene which expresses a protein capable of preventing HIV fusion into a cell or HIV replication; cloning the synthesized cDNA into a restriction site in a viral vector; and inserting an expression unit capable of down-regulating expression of an HIV co-receptor into a restriction site in the vector. The cDNA can be from any gene which expresses any of the protein fusion or replication inhibitors described herein. In some examples, the cDNA is a C46 cDNA. In another embodiment, the cDNA is a TRIM5α cDNA or a cyclophilin fusion thereof. The expression unit capable of down-regulating expression of a HIV co-receptor can be any of the inhibitory RNA molecules described herein, such as siRNA, shRNA, or antisense targeting the co-receptor. In one example, the expression unit is a shRNA targeting CCR5.

In some examples, an shRNA targeting an HIV-1 promoter is cloned into an existing vector, such as a vector expressing a shRNA targeting CCR5 and/or C46. In some examples, the existing plasmid is cut with a restriction enzyme. An in-fusion enzyme may then be used to fuse four or more sets of DNA segments by homologous recombination, the four or more sets of DNA sections engineered to contain approximately 15bp of homologous sequence, allowing for recombination of individual G Blocks. A first G Block, namely G Block 1, contains a U1 promoter sequence and half of the shPromA sequence. A second G Block, namely G Block 2, contains the other half of the shPromA sequence. Recombination is followed by transformation of E.coli with antibiotic selection.

RNA duplexes. Double-stranded RNA duplexes were designed to target the HIV-1 5'LTR region. RNA duplexes were synthesized by Invitrogen. Each siRNA construct was 19 bp in length and was designed with a 3'TT overhang.

Cell culture. Media and reagents for cell culture were purchased from Gibco. HeLa T4+ cells were grown in Dulbecco's modified Eagle's medium (DMEM) containing 10% fetal calf serum, 5 U/ml penicillin and 50 mg/mL streptomycin (supplemented DMEM) and incubated at 37°C in a humidified incubator with 5% CO2. HeLa T4+ cells were maintained under selection with G418 (500 µg/mL) and Hygromycin (300 µg/mL), respectively.

Live HIV-1 infection and siRNA transfection. Cell cultures for time course experiments using replication competent virus were seeded with 5 x104 HeLa-T4+ cells, incubated overnight and then transfected with 50 pM of the appropriate siRNA panel. The following day HeLa-T4+ siRNA-transfected cultures were infected with HIV-1 strain SF162, using 140 pg/µL. Supernatants were harvested over a time course of 15 days for analysis of virus production using the reverse transcriptase (RT) assay described below. Cultures for the ChIP experiment were seeded with 2 x105 HeLa-T4+ cells, incubated overnight and then infected with HIV-1 strain SF162 using 1000 pg/µL and the infection was allowed to proceed for 3 days. The infected cultures were then transfected with 300 pM siRNAs 143, 136, 205, PromA or mock-transfected for 48 h prior to harvest for the ChIP assay. Transfections were performed using RNAiMax (Invitrogen, Life Technologies) according to the manufacturer's instructions.

Viral Quantitation. Reverse transcriptase (RT) activity in culture supernatants was determined as described previously (Suzuki, K., Craddock, B. P., Okamoto, N., Kano, T., Steigbigel, R. T., J Virol Methods, 44: 189-98, 1993). HIV-1 mRNA was quantified using a real-time RT-PCR assay specific for HIV-gag as previously described (Suzuki et al., J RNAi Gene Silencing, 2005). Briefly, RT-PCR reactions were performed with SuperScript One-step RT-PCR (Invitrogen) using 0.4 µM of both sense and anti-sense primers, and 0.1 µM of sequence-specific fluorogenic Taqman probe. Standard curves were constructed using genomic HIV plasmid pNL4-3 for HIV-1 and a TA-cloned PCR fragment of beta-actin (Invitrogen, Mount Waverley, Australia). The primers and probes used are provided by SEQ ID NO: 30, SEQ ID NO: 31, SEQ ID NO: 32, SEQ ID NO: 33, SEQ ID NO: 34, and SEQ ID NO: 35.

### Drug treatments.

J-Lat 9.2 latent cell model cultures were treated with various concentrations of SAHA (0-100 uM), TNF (0-100 ng/mL) or a combination of SAHA/TNF (0-25 uM, 0-100 ng/mL) for 48 h. GFP expression was measured using flow cytometry (LSRFortessa-BD) to detect reactivation of integrated HIV-1 provirus and analyzed using FlowJo.

Chromatin Immunoprecipitation (ChIP) assays. HeLa T4+ cells (5 x 105) were seeded into T-25 flasks and 24 h later infected with HIV-1 strain SF162 as described above. At day 3 post-infection, the cultures were transfected with 300 pmol of each of siRNAs 143, 143T, 136, 205 and the current lead siRNA PromA, or mock-transfected as a control culture as described above. Following 48 h post-transfection, cultures were harvested for the ChIP assay using the EZ Magna A/G ChIP Kit (Millipore, Australia) following manufacturer's instructions. Pellets were sonicated for 20 minutes (lmin off, lmin on) in a COVARIS 5 sonicator at 5% Duty cycle: intensity 4, Burst/cycles 200, and protein isolated according to manufacturer's instructions. Immunoprecipitations were performed using 5 ug/ml of each antibody, specific to histones H3K27me3 (#17-622) and H3K9Ac (#17-658) obtained from Merck Millipore, for each 2x106 cell equivalents.

Statistical analysis. ChIP data were tested for significance using a non-parametric Mann-Whitney test and are given as mean ± SEM. A p-value of <0.05 was considered statistically significant. All analyses were performed using Graphpad Prism Version 6.0 (Graphpad Software, San Diego, CA).

### EXAMPLES

### Example 1. siRNAs targeting the U3 region can induce HIV-1 suppression.

Subtype B strain HIV-1SF162 was used to infect HeLa T4+ cultures and suprenantants were harvested over a prolonged time course and measured for reverse transcriptase (RT). To confirm whether a single mismatch can diminish the suppressive effect, siRNA 143T was included, containing a single T mismatch as it corresponds to a common variation at position 16 across HIV-1 subtypes A, D, F, G and U (Figure 5) and the HIV-1SF162 3'LTR GenBank sequence (accession number M65024.1). siRNAs, 143, 136T, 205S and PromA, potently suppressed HIV-1SF162 productive infection with >1000-fold reduction in RT activity compared to mock-transfected cells at day 15 post-infection. Interestingly, siRNA 143T suppressed virus infection similarly to siRNA 143 and PromA up to day 6 post-infection, but was unable to maintain virus suppression past this point. These data indicate a single target mismatch is sufficient to disrupt prolonged siRNA-induced HIV-1 virus suppression.

To determine sequence conservation of siRNAs 143, 136 and 205, a sequence alignment across HIV-1 subtypes A through G and U was performed and sequence logos were generated (Figure 3) (Crooks, Hon, Chandonia, & Brenner, 2004, Genome Res 14: 1188-90; Schneider, & Stephens, 1990, Nucleic Acids Res 18, 6097-100). For example, SiRNA 143 sequence conservation showed 94.9% median identity over 19 nucleotide positions with an identity range of 9/19 nucleotides having >95% conservation, 13/19 nucleotides having >90% conservation and 18/19 nucleotides having >80% conservation. As previously reported siPromA is highly conserved across all subtypes, with the exception of a one bp deletion in subtype C, and showed 98.4% median identity over 19 nucleotide positions (Figure 3).

### Example 2. Heterochromatin markers were observed in HIV-1 cultures suppressed by novel siRNA candidate 143 and PromA.

To determine whether the siRNA 143-mediated HIV-1 suppression was associated with heterochromatin marks as described for PromA, ChIP assays were performed for epigenetic modifications in the 5'LTR 48 h post-transfection. Significant increases were observed in H3K27me3; ∼10 fold in siRNA 143-transfected cultures (p <0.0001) and >5 fold in siRNA PromA-transfected cultures (p <0.0001) compared with the mock-transfected cultures (Figure 6a). Significant increases were also observed in H3K9me3; ∼5 fold in both siRNA 143- and siRNA PromA-transfected cultures (p <0.001) compared with the mock-transfected cultures (Figure 6b). Significant recruitment of Agol was observed in both siRNA 143- and siPromA-transfected cultures compared to mock-transfected cultures (Figure 6d). Significant reductions in the histone acetylation marker H3K9Ac were observed in siRNA 143- and PromA-transfected cultures (4-fold, and 6-fold, both p <0.05) (Figure 6e). Similarly, significant increases in H3K27me3 were reported in siRNA 136 and si205-transfected cultures compared to mock-transfected cultures (Figure 6f). Finally, significant decreases were reported in siRNA 136- and si205-transfected cultures compared to mock-transfected cultures (Figure 6g). Together, these data strongly suggest that the siRNA 143, 136 and 205 mimetics all silence HIV through TGS mechanisms.

### Example 3. shRNA targeting the 5'LTR diminish reactivation induced by drug treatments in latent HIV-1 J-Lat 9.2 cells.

To extend our study and investigate the effect of the siRNA143 target sequence in an HIV-1 latency model, we generated J-Lat 9.2 cells stably transduced with short hairpin sh143, shPromA or transduced with both shPromA and sh143 using separate lentivirus vectors. We then attempted to reactivate integrated latent HIV-1 in J-Lat 9.2 cells using SAHA, TNF or a combination of SAHA/TNF at various concentrations and measured GFP expression as a read out of HIV transcription, at 48 h. J-Lat 9.2 cells transduced with sh143, shPromA or dual sh143/shPromA were observed to be largely resistant to reactivation from SAHA, TNF or combinations of SAHA/TNF at physiological concentrations and showed low level reactivation even at supra-physiological drug concentrations, while those transduced with a shRNA control showed highly elevated GFP expression, indicating reactivation of latent HIV-1 infection (Figure 7). Importantly, the dual sh143/shPromA transduced J-Lat 9.2 cell line appeared to show a slightly better effect in protecting against reactivation stimuli. These data demonstrate that the HIV-1 latency model J-Lat 9.2 cells are largely resistant to reactivation by drug treatments when transduced with sh143 and/or shPromA.

### Example 4. Intracellular viral DNA and mRNA analysis with anti-HIV lentiviral transgenes - example of Cal-1 and TGS-inducing shRNA lentiviral constructs.

### (1) Cal-1 lentiviral vector based on HIV-1 entry inhibitors.

MOLT-4 based in-vitro infectious experiment. Three sets of samples were prepared based on MOLT-4 cells:
a. MOLT-4 cells only without any transduction of Cal-1 lentivirus;
b. A mixture of 80 % of MOLT-4 cells without any transduction of Cal-1 and 20% of Cal-1 transduced MOLT-4 cells. Degree of transduced cells was determined by flow analysis of C46 expression. This experimental setting is for monitoring the transgene effect of a mixed population of un-transduced MOLT-4 cells (80%) and Cal-1 transduced MOLT-4 cells (20%);
c. MOLT-4 Cal-1 transduced 100% with Cal-1 lentivirus as determined by C46 expression by Flow cytometry.

About 0.5 million of these cells are infected with HIV-1 BaL and samples were taken at day 4, 7, 10, 14 for analysis intracellular DNA and viral mRNA analysis.

Analysis of intracellular HIV-1 DNA (Figure 8a): HIV integrated DNA was analyzed using HIV-1 LTR region primer and probe set.

HIV-1 integrated DNA in the MOLT-4 cells was analyzed (Figure 8a). A significant increase in HIV-1 DNA levels was observed in MOLT-4 cells during the time course. This is indicative of a high level of HIV-1 integration in MOLT-4 cells.

The mixed MOLT-4 cell culture with 20% Cal-1 transduction showed HIV-1 DNA was detected on day 10 and day 14. However, the level of HIV-1 DNA observed was 10-50 fold lower as compared to un-transduced MOLT-4 cells. HIV-1 DNA was not detected in 100% Cal-1 transduced MOLT-4 cells.

In order to detect HIV-1 intercellular RNA level, HIV-1 specific LTR mRNA was analyzed (Figure 8b). Exceptionally elevated HIV-1 specific cellular associated HIV-1 LTR mRNA was detected in MOLT-4 cells (●) during the culture days.

In contrast, significant reduction of HIV-1 cellular associated HIV-1 LTR mRNA was observed in 100% Cal-1 transduced MOLT-4 cells (■). These levels were observed to be reduced greater than 1000-fold. The level of reduction in this experimental group (10-100 fold) in HIV-1 cellular associated HIV-1 LTR mRNA in the mixed MOLT-4 cells with 20% transduced efficiency of Cal-1 (▲) is indicative of partial protection from HIV infection. These observations demonstrate the Cal-1 lentivirus vector is able to induce greater than 1000 times reduction of HIV transcription in MOLT-4 cells.

Despite HIV-1 DNA level not being detected in Cal-1 transduced MOLT-4 cells, HIV-1 cellular associated LTR-mRNA was still detectable (■) in Figure 8b. This is an indication that a very small level of RNA transcription was taking place from a very small population of HIV-1 integrated DNA in MOLT-4 cells.

An estimated cell number per this DNA PCR was about 1-2 x 10e5 cell equivalent amount DNA per one PCR reaction. Therefore, HIV-1 detection sensitivity in this assay format is 2 copies per 1-2 x 10e5 cells. If the HIV-1 DNA integrated frequency were less than 2 copy per 1-2 x 10e5 cells, the assay would not be capable of detecting HIV-1 DNA in this format.

It is possible that the HIV-1 LTR-mRNA levels observed were the result of a very small fraction of MOLT-4 cells, which failed to be transduced with Cal-1.

### (2) TGS-inducing shRNA lentiviral vector.

PM-1 based in-vitro infectious experiment. Three sets of samples were prepared based on the PM-1 CD4+ T-cell line:
a. PM-1 cells without any transduction of shRNA lentivirus;
b. PM-1 cells transduced with shPromA (shRNA targeting HIV-1 promoter region (detail of shPromA construct was described: Suzuki K, et.al, Mol Ther Nucleic Acids 2013, 2:e137);
c. PM-1 cells transduced with shPromA-Scrambled control.

About 0.5 million of these cells were infected with HIV-1 strain JR-FL and samples were taken at day 10 for analysis intracellular DNA and viral mRNA analysis.

HIV-1 integrated DNA in the PM-1 cells was analyzed (Figure 9a). The three sets of experimental conditions contained relatively similar amounts of HIV-1 DNA.

In order to detect HIV-1 intercellular RNA level, HIV-1 specific gag mRNA was analyzed (Figure 9b). Despite the presence of HIV-1 DNA in shPromA transduced PM-1 cells (Figure 9a), HIV-1 transcription was greatly suppressed. Specifically, around 1000-fold reduction was observed as compared to the un-transduced PM-1 mock infection control and shPromA-Scrambled control. These observations are an indication that shPromA is able to induce around 1000 times reduction of HIV-1 transcription using the PM-1 cell model.

Based on the above data, a combination approach comprising Cal-1 and TSG-inducing shRNA is predicted to generate greater than 106 order of suppression in transduced T-cell lines compared to un-transduced mock infection. Therefore, we expect to observe a complete suppression of expression of HIV-1 transcription by this triple and/or quadruple combination anti-HIV lentivirus vector system.

### Example 5

Applicants believe that triple or quadruple combination vectors according to the present disclosure are at least as safe and efficacious as dual vector constructions, such as Cal-1, described herein. Applicants have shown that autologous transplantation utilizing Cal-1 lentiviral vectors is safe, and mediates stable gene marking in macaque peripheral blood following a single transplant using myeloablative conditioning and without methods of selection for gene-modified cells. Applicants have also shown that animals transplanted with Cal-1 transduced CD34+ cells exhibit long-term, multi-lineage engraftment of gene-marked cells, and that these cells are capable of resisting SHIV challenge ex vivo and in vivo. Indeed, ex vivo assays show that Cal-1 transduction efficiency of CD34+ cells correlates with gene marking levels in peripheral blood and tissues of transplanted animals measured prior to infection, that CD4+ cells from Cal-1-transplanted animals are resistant to ex vivo SHIV infection, and that gene-marked cells undergo virus-dependent positive selection. Applicants have also shown in subset sorting experiments that there exists a preferential increase in marking in SHIV-susceptible subsets, namely in the T-cell compartment.

Based on at least these findings, Applicants believe that the incorporation of one or more transcriptional gene silencing elements into the existing Cal-1 vector will yield a triple or quadruple construct vector that is at least as safe and at least as efficacious as Cal-1 (or at least as safe and efficacious as a vector comprising one or more transcriptional gene silencing elements). As such, the combined potency and toxicity of the triple and quadruple combination lentiviral (LV) constructs (see Figure 10) will be tested in a series of in vitro and in vivo experiments, with the specific aim of demonstrating whether combining the effect of the expression vector comprising shRNA against CCR5 and/or the C46 fusion inhibitor) with the effect of shRNAs inducing transcriptional gene silencing (TGS) from the same lentiviral vector is complementary or even synergistic in terms of control of the viral reservoir. There is now increasing evidence that such multiplexed constructs can efficiently express and allow processing of multiple shRNAs using combinations of H1, U6 and U1 promoters.
2a) Once the lentiviral constructs of the present disclosure are synthesized, we will demonstrate that there is effective and expected levels of expression of each of the TGS inducing and CCR5 inhibiting shRNAs and that they are as effectively expressed and processed.
2b) Applicants will demonstrate lentiviral constructs of the present disclosure can induce TGS as efficiently as the transcriptional gene silencing element alone by monitoring the dynamics of infection with a CXCR4 tropic virus (NL4.3) and a VZV-G pseudotyped virus. Both these viruses will evade the CCR5 shRNA and the latter will evade C46, allowing the degree of silencing achieved via shRNA-induced TGS expressed from constructs of the present disclosure to be assessed in vitro independent of the effects of the other nucleic acid components components (e.g. directed to CCR4 and/or C46, etc.). In parallel, using a β-lactamase (BLAM) reporter assay we will demonstrate inhibition of entry of a CCR5 using virus to demonstrate that constructs of the present disclosure have equivalent efficacy to those constructs described in U.S. Publication No. 2012/0201794. This will be confirmed by quantifying relative lentiviral marking and HIV-1 DNA in these cultures. These experiments will demonstrate the equivalence of expression and efficacy of each individual shRNA when delivered by standard (single/dual agent construct) or complex (triple/quadruple agent) lentiviral constructs.
2c) Once this has been demonstrated, experiments similar to those described in PCT/AU2015/050507 (published as WO 2016/029275 A1) will be conducted to compare the efficacy and extent of off-target effects between (1) the expression vectors/constructions as described in U.S. Publication No. 2012/0201794, (2) the lead TGS candidate from PCT/AU2015/050507 (published as WO 2016/029275 A1), and (3) the constructs of the present disclosure. These experiments will demonstrate whether any of constructs of the present disclosure have greater effects than either those described in U.S. Publication No. 2012/0201794 or those described in PCT/AU2015/050507 (published as WO 2016/029275 A1), and particularly, because they act at different stages of the life cycle, whether the effect is additive, or even synergistic.
2d) Once in vitro experiments have demonstrated at least additive effects of one of LV of the present invention, they will be utilized for in vivo assessment of efficacy in the BLT mouse model as described above in a four arm experiment comparing its efficacy directly to the lead TGS LV construct from PCT/AU2015/050507 (published as WO 2016/029275 A1), U.S. Publication No. 2012/0201794, and empty vector. The primary end point will be the size of the integrated HIV reservoir in CD4+T cells from the spleen. Secondary endpoints will include pVL and CD4+ T cell counts.
2e) Once the potential advantage of the LV construct of the present disclosure is demonstrated in vivo, in addition to CD34+ HSC transduction and challenge, we will reconstitute BLT mice with transduced CD4+ T cells obtained from a HIV infected donor to demonstrate that in the presence of an established virus reservoir, a lead LV construct of the present disclosure can effectively silence pre-existing integrated virus.

Outcomes: We expect that LV construct viii) will best fulfil the outcomes of a)-c) and that due to multiple virus targets the suppressive effect will be at least additive. In vivo studies are expected to confirm more potent and more prolonged control of the viral reservoir, pVL virus and maintenance of CD4+ T cells counts, both in the periphery and tissues. Preliminary toxicity data will also be obtained.

### STATEMENT OF INDUSTRIAL APPLICABILITY

The present disclosure has applicability in the field of medicine, e.g. gene therapy.

## Claims

1. An expression vector comprising (a) at least one nucleic acid sequence encoding a transcriptional gene silencing element; and (b) at least two other nucleic acid sequences selected from the group consisting of a nucleic acid sequence that encodes a nucleic acid molecule that inhibits an HIV co-receptor; a nucleic acid sequence that encodes an HIV fusion inhibitor protein; a nucleic acid sequence encoding an inhibitor of HIV replication; and a nucleic acid sequence encoding an inhibitor of viral entry; wherein the at least one nucleic acid sequence encoding a transcriptional gene silencing element is selected from the group consisting of (i) a silencing nucleic acid which targets a sequence from about position 143 to about position 161 of a 5' LTR of HIV-1, (ii) a silencing nucleic acid which targets a sequence from about position 136 to about position 154 of the 5' LTR of HIV-1, (iii) a silencing nucleic acid which targets a sequence from about position 205 to about position 223 of the 5' LTR of HIV-1; and (iv) a silencing nucleic acid which targets a sequence from about position 350 to about position 368 of the 5' LTR of HIV-1.

2. The expression vector of claim 1, wherein the at least one nucleic acid sequence encoding a transcriptional gene silencing element is selected from the group consisting of (i) a silencing nucleic acid which targets the sequence of SEQ ID NO: 1, (ii) a silencing nucleic acid which targets the sequence of SEQ ID NO: 9, (iii) a silencing nucleic acid which targets the sequence of SEQ ID NO: 17; and (iv) a silencing nucleic acid which targets the sequence of SEQ ID NO: 36.

3. The expression vector of claim 1, wherein the at least one nucleic acid sequence encoding a transcriptional gene silencing element is an RNA duplex comprising a sense strand and an antisense strand, wherein the sense strand comprises a sequence having at least 95% identity to one of SEQ ID NO: 6, the sequence of SEQ ID NO: 14, the sequence of SEQ ID NO: 22, or the sequence of SEQ ID NO: 40.

4. The expression vector of claim 1, wherein the at least one nucleic acid sequence encoding a transcriptional gene silencing element is an shRNA selected from the group consisting of (i) an shRNA having the sequence of SEQ ID NO: 8; (ii) an shRNA having the sequence of SEQ ID NO: 16; (iii) an shRNA having the sequence of SEQ ID NO: 24; and (iv) an shRNA having the sequence of SEQ ID NO: 42.

5. The expression vector of any of the preceding claims, wherein the protein that inhibits HIV replication is selected from the group consisting of human TRIM5α, rhesus TRIM5α, chimeric TRIM5α, a human TRIM5-cyclophilin fusion protein, cyclophilin, E3 ubiquitin, APOBEC3G, and bone marrow stromal cell antigen 2 (BST-2).

6. The expression vector of any of claims 1 to 5, wherein the at least two other nucleic acid sequences comprise a nucleic acid sequence that encodes a nucleic acid molecule that inhibits an HIV co-receptor; and a nucleic acid sequence that encodes an HIV fusion inhibitor protein.

7. The expression vector of claim 1, wherein the nucleic acid sequence that encodes the nucleic acid molecule that inhibits an HIV co-receptor has a sequence of SEQ ID NO: 25.

8. The expression vector of claim 1, wherein the nucleic acid sequence that encodes the HIV fusion inhibitor protein has the sequence of SEQ ID NO: 26.

9. The expression vector of any of the preceding claims, wherein the viral vector is a self-inactivating lentiviral vector or a retroviral vector.

10. The expression vector of any of the preceding claims, further comprising a fourth nucleic acid sequence encoding another silencing nucleic acid that targets a sequence within the 5' LTR region of HIV, wherein the fourth nucleic acid is selected from the group consisting of (i) a silencing nucleic acid which targets a sequence from about position 143 to about position 161 of the 5' LTR of HIV-1, (ii) a silencing nucleic acid which targets a sequence from about position 136 to about position 154 of the 5' LTR of HIV-1, (iii) a silencing nucleic acid which targets a sequence from about position 205 to about position 223 of the 5' LTR of HIV-1; and (iv) a silencing nucleic acid which targets a sequence from about position 350 to about position 368 of the 5' LTR of HIV-1; and wherein the fourth nucleic acid sequence is different than the third nucleic acid sequence.

11. An in vitro method for treating human hematopoietic stem cells infected with HIV, comprising providing hematopoietic stem cells extracted from circulating blood or bone marrow of a host patient; and treating the hematopoietic stem cells with the expression vector of any of claims 1 to 10.

12. An expression vector of any of claims 1 to 10 for use in the treatment of HIV.

13. A host cell transduced by the expression vector of any of claims 1 to 10.

14. A composition comprising the expression vector of any of claims 1 to 10; and a pharmaceutically acceptable carrier or excipient.

## Patentansprüche

1. Expressionsvektor, aufweisend (a) mindestens eine Nukleinsäuresequenz, die ein transkriptionelles Gen-Silencing-Element codiert; und (b) mindestens zwei andere Nukleinsäuresequenzen, ausgewählt aus der Gruppe bestehend aus einer Nukleinsäuresequenz, die ein Nukleinsäuremolekül codiert, das einen HIV-Co-Rezeptor hemmt; einer Nukleinsäuresequenz, die ein HIV-Fusionshemmerprotein codiert; einer Nukleinsäuresequenz, die einen Hemmstoff der HIV-Replikation codiert; und einer Nukleinsäuresequenz, die einen Hemmstoff des Viruseintritts codiert; wobei die mindestens eine Nukleinsäuresequenz, die ein transkriptionelles Gen-Silencing-Element codiert, ausgewählt ist aus der Gruppe bestehend aus (i) einer Silencing-Nukleinsäure, die auf eine Sequenz von etwa Position 143 bis etwa Position 161 einer 5'-LTR von HIV-1 abzielt, (ii) einer Silencing-Nukleinsäure, die auf eine Sequenz von etwa Position 136 bis etwa Position 154 der 5'-LTR von HIV-1 abzielt, (iii) einer Silencing-Nukleinsäure, die auf eine Sequenz von etwa Position 205 bis etwa Position 223 der 5'-LTR von HIV-1 abzielt; und (iv) einer Silencing-Nukleinsäure, die auf eine Sequenz von etwa Position 350 bis etwa Position 368 der 5'-LTR von HIV-1 abzielt.

2. Expressionsvektor nach Anspruch 1, bei welchem die mindestens eine Nukleinsäuresequenz, die ein transkriptionelles Gen-Silencing-Element codiert, ausgewählt ist aus der Gruppe bestehend aus (i) einer Silencing-Nukleinsäure, die auf die Sequenz von SEQ ID NO: 1 abzielt, (ii) einer Silencing-Nukleinsäure, die auf die Sequenz von SEQ ID NO: 9 abzielt, (iii) einer Silencing-Nukleinsäure, die auf die Sequenz von SEQ ID NO: 17 abzielt, und (iv) einer Silencing-Nukleinsäure, die auf die Sequenz von SEQ ID NO: 36 abzielt.

3. Expressionsvektor nach Anspruch 1, bei welchem die mindestens eine Nukleinsäuresequenz, die ein transkriptionelles Gen-Silencing-Element codiert, ein RNA-Duplex mit einem Sense-Strang und einem Antisense-Strang ist, wobei der Sense-Strang eine Sequenz mit wenigstens 95% Identität zu einer der Sequenz von SEQ ID NO: 6, der Sequenz von SEQ ID NO: 14, der Sequenz von SEQ ID NO: 22 oder der Sequenz von SEQ ID NO: 40 aufweist.

4. Expressionsvektor nach Anspruch 1, bei welchem die mindestens eine Nukleinsäuresequenz, die ein transkriptionelles Gen-Silencing-Element codiert, eine shRNA ist, ausgewählt aus der Gruppe bestehend aus (i) einer shRNA mit der Sequenz von SEQ ID NO: 8; (ii) einer shRNA mit der Sequenz von SEQ ID NO: 16; (iii) einer shRNA mit der Sequenz von SEQ ID NO: 24; und (iv) einer shRNA mit der Sequenz von SEQ ID NO: 42.

5. Expressionsvektor nach einem der vorhergehenden Ansprüche, bei welchem das Protein, das die HIV-Replikation hemmt, ausgewählt ist aus der Gruppe bestehend aus menschlichem TRIM5α, Rhesus-TRIM5α, chimärem TRIM5α, menschlichem TRIM5-Cyklophilin-Fusionsprotein, Cyklophilin, E3-Ubiquitin, APOBEC3G und Knochenmark-Stromazellen-Antigen 2 (BST-2).

6. Expressionsvektor nach einem der Ansprüche 1 bis 5, bei welchem die mindestens zwei anderen Nukleinsäuresequenzen eine Nukleinsäuresequenz, die ein Nukleinsäuremolekül codiert, das einen HIV-Co-Rezeptor hemmt; und eine Nukleinsäuresequenz, die ein HIV-Fusionshemmer-Protein codiert, aufweisen.

7. Expressionsvektor nach Anspruch 1, bei welchem die Nukleinsäuresequenz, die das Nukleinsäuremolekül codiert, das einen HIV-Co-Rezeptor hemmt, eine Sequenz von SEQ ID NO: 25 hat.

8. Expressionsvektor nach Anspruch 1, bei welchem die Nukleinsäuresequenz, die das HIV-Fusionshemmer-Protein codiert, die Sequenz von SEQ ID NO: 26 hat.

9. Expressionsvektor nach einem der vorhergehenden Ansprüche, bei welchem der virale Vektor ein selbstinaktivierender lentiviraler Vektor oder ein retroviraler Vektor ist.

10. Expressionsvektor nach einem der vorhergehenden Ansprüche, ferner aufweisend eine vierte Nukleinsäuresequenz, die eine weitere Silencing-Nukleinsäure codiert, die auf eine Sequenz im 5'-LTR-Bereich von HV abzielt, wobei die vierte Nukleinsäure ausgewählt ist aus der Gruppe bestehend aus (i) einer Silencing-Nukleinsäure, die auf eine Sequenz von etwa Position 143 bis etwa Position 161 der 5'-LTR von HIV-1 abzielt, (ii) einer Silencing-Nukleinsäure, die auf eine Sequenz von etwa Position 136 bis etwa Position 154 der 5'-LTR von HIV-1 abzielt, (iii) einer Silencing-Nukleinsäure, die auf eine Sequenz von etwa Position 205 bis etwa Position 223 der 5'-LTR von HIV-1 abzielt, und (iv) einer Silencing-Nukleinsäure, die auf eine Sequenz von etwa Position 350 bis etwa Position 368 der 5'-LTR von HIV-1 abzielt; und wobei die vierte Nukleinsäuresequenz anders als die dritte Nukleinsäuresequenz ist.

11. In-Vitro-Verfahren zum Behandeln von menschlichen hämatopoetischen Stammzellen, die mit HIV infiziert sind, aufweisend ein Vorlegen von hämatopoetischen Stammzellen, die aus einem zirkulierenden Blut oder einem Knochenmark eines Wirts / Patienten extrahiert werden; und ein Behandeln der hämatopoetischen Stammzellen mit dem Expressionsvektor nach einem der Ansprüche 1 bis 10.

12. Expressionsvektor nach einem der Ansprüche 1 bis 10 zur Verwendung in der Behandlung von HIV.

13. Wirtszelle, die durch den Expressionsvektor nach einem der Ansprüche 1 bis 10 umgewandelt ist.

14. Zusammensetzung, aufweisend den Expressionsvektor nach einem der Ansprüche 1 bis 10; und einen pharmazeutisch verträglichen Träger oder Trägerstoff.

## Revendications

1. Vecteur d'expression comprenant (a) au moins une séquence d'acide nucléique codant pour un élément de silençage génique transcriptionnel ; et (b) au moins deux autres séquences d'acide nucléique sélectionnées dans le groupe consistant en une séquence d'acide nucléique codant pour une molécule d'acide nucléique qui inhibe un corécepteur du VIH ; une séquence d'acide nucléique codant pour une protéine inhibitrice de la fusion du VIH ; une séquence d'acide nucléique codant pour un inhibiteur de la réplication du VIH ; et une séquence d'acide nucléique codant pour un inhibiteur de l'entrée virale ; dans lequel ladite au moins une séquence d'acide nucléique codant pour un élément de silençage génique transcriptionnel est sélectionnée dans le groupe consistant en (i) un acide nucléique de silençage qui cible une séquence d'environ la position 143 à environ la position 161 d'un LTR 5' du VIH-1 ; (ii) un acide nucléique de silençage qui cible une séquence d'environ la position 136 à environ la position 154 du LTR 5' du VIH-1 ; (iii) un acide nucléique de silençage qui cible une séquence d'environ la position 205 à environ la position 223 du LTR 5' du VIH-1 ; et (iv) un acide nucléique de silençage qui cible une séquence allant d'environ la position 350 à environ la position 368 du LTR 5' du VIH-1.

2. Vecteur d'expression selon la revendication 1, dans lequel ladite au moins une séquence d'acide nucléique codant pour un élément de silençage génique transcriptionnel est sélectionnée dans le groupe consistant en (i) un acide nucléique de silençage qui cible la séquence de SEQ ID NO : 1 ; (ii) un acide nucléique de silençage qui cible la séquence de SEQ ID NO : 9 ; (iii) un acide nucléique de silençage qui cible la séquence de SEQ ID NO : 17 ; et (iv) un acide nucléique de silençage qui cible la séquence de SEQ ID NO : 36.

3. Vecteur d'expression selon la revendication 1, dans lequel ladite au moins une séquence d'acide nucléique codant pour un élément de silençage génique transcriptionnel est un duplex d'ARN comprenant un brin sens et un brin antisens, dans lequel le brin sens comprend une séquence ayant au moins 95 % d'identité avec l'une des séquences de SEQ ID NO : 6, SEQ ID NO : 14, SEQ ID NO : 22, ou SEQ ID NO : 40.

4. Vecteur d'expression selon la revendication 1, dans lequel ladite au moins une séquence d'acide nucléique codant pour un élément de silençage génique transcriptionnel est un shARN sélectionné dans le groupe consistant en (i) un shARN ayant la séquence de SEQ ID NO : 8 ; (ii) un shARN ayant la séquence de SEQ ID NO : 16 ; (iii) un shARN ayant la séquence de SEQ ID NO : 24 ; et (iv) un shARN ayant la séquence de SEQ ID NO : 42.

5. Vecteur d'expression selon l'une quelconque des revendications précédentes, dans lequel la protéine qui inhibe la réplication du HIV est sélectionnée dans le groupe consistant en le TRIM5α humain, le TRIM5α du rhésus, le TRIM5α chimérique, une protéine de fusion entre le TRIM5 humain et la cyclophiline, la cyclophiline, l'ubiquitine E3, l'APOBEC3G, et l'antigène 2 des cellules stromales de la moelle osseuse (BST-2).

6. Vecteur d'expression selon l'une quelconque des revendications 1 à 5, dans lequel lesdites au moins deux autres séquences d'acide nucléique comprennent une séquence d'acide nucléique qui code pour une molécule d'acide nucléique qui inhibe un corécepteur HIV, et une séquence d'acide nucléique qui code pour une protéine inhibitrice de la fusion du HIV.

7. Vecteur d'expression selon la revendication 1, dans lequel la séquence d'acide nucléique qui code pour la molécule d'acide nucléique qui inhibe un corécepteur du HIV, a une séquence de SEQ ID NO : 25.

8. Vecteur d'expression selon la revendication 1, dans lequel la séquence d'acide nucléique qui code pour la protéine inhibitrice de la fusion du HIV, a la séquence de SEQ ID NO : 26.

9. Vecteur d'expression selon l'une quelconque des revendications précédentes, dans lequel le vecteur viral est un vecteur lentiviral auto-inactivant ou un vecteur rétroviral.

10. Vecteur d'expression selon l'une quelconque des revendications précédentes, comprenant en outre une quatrième séquence d'acide nucléique codant pour un autre acide nucléique de silençage qui cible une séquence dans la région LTR 5' du VIH, dans lequel le quatrième acide nucléique est sélectionné dans le groupe consistant en (i) un acide nucléique de silençage qui cible une séquence d'environ la position 143 à environ la position 161 du LTR 5' du VIH-1 ; (ii) un acide nucléique de silençage qui cible une séquence d'environ la position 136 à environ la position 154 du LTR 5' du VIH-1 ; (iii) un acide nucléique de silençage qui cible une séquence d'environ la position 205 à environ la position 223 du LTR 5' du VIH-1 ; et (iv) un acide nucléique de silençage qui cible une séquence d'environ la position 350 à environ la position 368 du LTR 5' du VIH-1, et dans lequel la quatrième séquence d'acide nucléique est différente de la troisième séquence d'acide nucléique.

11. Procédé in vitro de traitement de cellules souches hématopoïétiques humaines infectées par le VIH, consistant à fournir des cellules souches hématopoïétiques extraites du sang en circulation ou de la moelle osseuse d'un patient hôte, et à traiter les cellules souches hématopoïétiques avec le vecteur d'expression selon l'une quelconque des revendications 1 à 10.

12. Vecteur d'expression selon l'une quelconque des revendications 1 à 10 à utiliser dans le traitement du HIV.

13. cellule hôte transduite par le vecteur d'expression selon l'une quelconque des revendications 1 à 10.

14. Composition comprenant le vecteur d'expression selon l'une quelconque des revendications 1 à 10, et un support ou excipient acceptable sur le plan pharmaceutique.


### SEQUENCE LISTING

<110> CALIMMUNE, INC.
   CALIMMUNE AUSTRALIA PTY LTD
   NEWSOUTH INNOVATIONS PTY LIMITED
   ST. VINCENT'S HOSPITAL SYDNEY
   SYMONDS, Geoffrey Phillip
   SUZUKI, Kazuo
   KELLEHER, Anthony Dominic
   AHLENSTIEL, Chantelle Lisa Evelyn
<120> GENE THERAPEUTIC FOR THE TREATMENT OF HIV AND USES THEREOF
<130> Cal-002WO
<150> US 62/163, 332
   <151> 2015-05-18
<160> 43
<170> PatentIn version 3.5
<210> 1
   <211> 19
   <212> DNA
   <213> HIV-1
<220>
   <221> misc_feature
   <222> (1)..(19)
   <223> 143 to 161 5' LTR U3
<400> 1
   gctagtacca gttgagcca 19
<210> 2
   <211> 19
   <212> DNA
   <213> HIV-1
<220>
   <221> misc_feature
   <222> (1)..(19)
   <223> strain BaL, subtypes B, D, F, H, J, K (143 to 161 5' LTR U3)
<400> 2
   gctagtacca gttgagcca 19
<210> 3
   <211> 19
   <212> DNA
   <213> HIV-1
<220>
   <221> misc_feature
   <222> (1)..(19)
   <223> subtype A (143 to 161 5' LTR U3)
<400> 3
   gctagtacca gttgatcca 19
<210> 4
   <211> 19
   <212> DNA
   <213> HIV-1
<220>
   <221> misc_feature
   <222> (1)..(19)
   <223> subtype C (143 to 161 5' LTR U3)
<400> 4
   gctagtgcca gttgaccca 19
<210> 5
   <211> 19
   <212> DNA
   <213> HIV-1
<220>
   <221> misc_feature
   <222> (1)..(19)
   <223> subtype G (143 to 161 5' LTR U3)
<400> 5
   gctagtacca gtggaccca 19
<210> 6
   <211> 19
   <212> RNA
   <213> Artificial Sequence
<220>
   <223> Synthetic: RNA duplex sense strand (143 to 161 5' LTR U3)
<400> 6
   gcuaguacca guugagcca 19
<210> 7
   <211> 19
   <212> RNA
   <213> Artificial Sequence
<220>
   <223> Synthetic: RNA duplex antisense strand (143 to 161 5' LTR U3)
<400> 7
   uggcucaacu gguacuagc 19
<210> 8
   <211> 57
   <212> RNA
   <213> Artificial Sequence
<220>
   <223> Synthetic: shRNA (143 to 161 5' LTR U3)
<400> 8
   gcuaguacca guugagccac ugugaagcca cagaugggug gcucaacugg uacuagc 57
<210> 9
   <211> 19
   <212> DNA
   <213> HIV-1
<220>
   <221> misc_feature
   <222> (1)..(19)
   <223> 136 to 154 5' LTR U3
<400> 9
   gctacaagct agtaccagt 19
<210> 10
   <211> 19
   <212> DNA
   <213> HIV-1
<220>
   <221> misc_feature
   <222> (1)..(19)
   <223> strain SF162, subtypes A, B, F, H, J, K (136 to 154 5' LTR U3)
<400> 10
   gcttcaagct agtaccagt 19
<210> 11
   <211> 19
   <212> DNA
   <213> HIV-1
<220>
   <221> misc_feature
   <222> (1)..(19)
   <223> subtype C (136 to 154 5' LTR U3)
<400> 11
   gcttcaagct agtgccagt 19
<210> 12
   <211> 19
   <212> DNA
   <213> HIV-1
<220>
   <221> misc_feature
   <222> (1)..(19)
   <223> subtype D (136 to 154 5' LTR U3)
<400> 12
   gcttcgagct agtaccagt 19
<210> 13
   <211> 19
   <212> DNA
   <213> HIV-1
<220>
   <221> misc_feature
   <222> (1)..(19)
   <223> subtype G (136 to 154 5' LTR U3)
<400> 13
   gcttcaaact agtaccaat 19
<210> 14
   <211> 19
   <212> RNA
   <213> Artificial Sequence
<220>
   <223> Synthetic: RNA duplex sense strand (136 to 154 5' LTR U3)
<400> 14
   gcuacaagcu aguaccagu 19
<210> 15
   <211> 19
   <212> RNA
   <213> Artificial Sequence
<220>
   <223> Synthetic: RNA duplex antisense strand (136 to 154 5' LTR U3)
<400> 15
   acugguacua gcuuguagc 19
<210> 16
   <211> 57
   <212> RNA
   <213> Artificial Sequence
<220>
   <223> Synthetic: shRNA (136 to 154 5' LTR U3)
<400> 16
   gcuucaagcu aguaccaguc ugugaagcca cagaugggac ugguacuagc uugaagc 57
<210> 17
   <211> 19
   <212> DNA
   <213> HIV-1
<220>
   <221> misc_feature
   <222> (1)..(19)
   <223> 205 to 223 5' LTR U3
<400> 17
   acaccctatg agcctgcat 19
<210> 18
   <211> 19
   <212> DNA
   <213> HIV-1
<220>
   <221> misc_feature
   <222> (1)..(19)
   <223> strain SF162, subtypes A, B, H, J, K (205 to 223 5' LTR U3)
<400> 18
   acaccctatg agcctgcat 19
<210> 19
   <211> 19
   <212> DNA
   <213> HIV-1
<220>
   <221> misc_feature
   <222> (1)..(19)
   <223> subtype C and D (205 to 223 5' LTR U3)
<400> 19
   acaccctatg agccagcat 19
<210> 20
   <211> 19
   <212> DNA
   <213> HIV-1
<220>
   <221> misc_feature
   <222> (1)..(19)
   <223> Subtype F (205 to 223 5' LTR U3)
<400> 20
   acaccccatg agccaacat 19
<210> 21
   <211> 19
   <212> DNA
   <213> HIV-1
<220>
   <221> misc_feature
   <222> (1)..(19)
   <223> Subtype G (205 to 223 5' LTR U3)
<400> 21
   acaccccatc tgccagcat 19
<210> 22
   <211> 19
   <212> RNA
   <213> Artificial Sequence
<220>
   <223> Synthetic: RNA duplex sense strand (205 to 223 5' LTR U3)
<400> 22
   acacccugug agccugcau 19
<210> 23
   <211> 19
   <212> RNA
   <213> Artificial Sequence
<220>
   <223> Synthetic: RNA duplex antisense strand (205 to 223 5' LTR U3)
<400> 23
   augcaggcuc acagggugu 19
<210> 24
   <211> 57
   <212> RNA
   <213> Artificial Sequence
<220>
   <223> Synthetic: shRNA (205 to 223 5' LTR U3)
<400> 24
   acacccuaug agccugcauc ugugaagcca cagaugggau gcaggcucau agggugu 57
<210> 25
   <211> 51
   <212> RNA
   <213> Unknown
<220>
   <223> shRNA targeting CCR5 receptor
<400> 25
   gagcaagcuc aguuuacacc uuguccgacg guguaaacug agcuugcucu u 51
<210> 26
   <211> 135
   <212> PRT
   <213> Artificial Sequence
<220>
   <223> Synthetic: C46 HIV fusion inhibitor
<400> 26
<210> 27
   <211> 408
   <212> DNA
   <213> Artificial Sequence
<220>
   <223> Synthetic: C46 HIV fusion inhibitor
<400> 27
   atgggagcag gagcaaccgg aagggcaatg gacggaccac ggctgctgct gctgctgctg 60
   ctgggcgtga gcctgggcgg cgcccggagc tggatggagt gggaccggga gatcaacaac 120
   tacaccagcc tgatccacag cctgatcgag gagagccaga accagcagga gaagaacgag 180
   caggagctgc tggagctgga caagtgggcc agcctgtgga actggttccg gagcgagcgg 240
   aagtgctgcg tggagtgccc accatgccca gcaccaccag tggcaggacc cctgatcgca 300
   ctggtgacca gcggagccct gctggccgtg ctgggcatca caggctactt cctgatgaac 360
   cggcggagct ggagccccac cggcgagcgg ctggagctgg agccctga 408
<210> 28
   <211> 493
   <212> PRT
   <213> Homo sapiens
<220>
   <221> misc_feature
   <222> (1)..(493)
   <223> human TRIM5-alpha protein
<400> 28
   Met Ala Ser Gly Ile Leu Val Asn Val Lys Glu Glu Val Thr Cys Pro
   1               5                   10                  15
   Ile Cys Leu Glu Leu Leu Thr Gln Pro Leu Ser Leu Asp Cys Gly His
               20                  25                  30
   Ser Phe Cys Gln Ala Cys Leu Thr Ala Asn His Lys Lys Ser Met Leu
           35                  40                  45
   Asp Lys Gly Glu Ser Ser Cys Pro Val Cys Arg Ile Ser Tyr Gln Pro
       50                  55                  60
   Glu Asn Ile Arg Pro Asn Arg His Val Ala Asn Ile Val Glu Gly Leu
   65                  70                  75                  80
   Arg Glu Val Lys Leu Ser Pro Glu Gly Gln Lys Val Asp His Cys Ala
                   85                  90                  95
   Arg His Gly Glu Lys Leu Leu Leu Phe Cys Gln Glu Asp Gly Lys Val
               100                 105                 110
   Ile Cys Trp Leu Cys Glu Arg Ser Gln Glu His Arg Gly His His Thr
           115                 120                 125
   Phe Leu Thr Glu Glu Val Ala Arg Glu Tyr Gln Val Lys Leu Gln Ala
       130                 135                 140
   Ala Leu Glu Met Leu Arg Gln Lys Gln Gln Glu Ala Glu Glu Leu Glu
   145                 150                 155                 160
   Ala Asp Ile Arg Glu Glu Lys Ala Ser Trp Lys Thr Gln Ile Gln Tyr
                   165                 170                 175
   Asp Lys Thr Asn Val Leu Ala Asp Phe Glu Gln Leu Arg Asp Ile Leu
               180                 185                 190
   Asp Trp Glu Glu Ser Asn Glu Leu Gln Asn Leu Glu Lys Glu Glu Glu
           195                 200                 205
   Asp Ile Leu Lys Ser Leu Thr Asn Ser Glu Thr Glu Met Val Gln Gln
       210                 215                 220
   Thr Gln Ser Leu Arg Glu Leu Ile Ser Asp Leu Glu His Arg Leu Gln
   225                 230                 235                 240
   Gly Ser Val Met Glu Leu Leu Gln Gly Val Asp Gly Val Ile Lys Arg
                   245                 250                 255
   Thr Glu Asn Val Thr Leu Lys Lys Pro Glu Thr Phe Pro Lys Asn Gln
               260                 265                 270
   Arg Arg Val Phe Arg Ala Pro Asp Leu Lys Gly Met Leu Glu Val Phe
           275                 280                 285
   Arg Glu Leu Thr Asp Val Arg Arg Tyr Trp Val Asp Val Thr Val Ala
       290                 295                 300
   Pro Asn Asn Ile Ser Cys Ala Val Ile Ser Glu Asp Lys Arg Gln Val
   305                 310                 315                 320
   Ser Ser Pro Lys Pro Gln Ile Ile Tyr Gly Ala Arg Gly Thr Arg Tyr
                   325                 330                 335
   Gln Thr Phe Val Asn Phe Asn Tyr Cys Thr Gly Ile Leu Gly Ser Gln
               340                 345                 350
   Ser Ile Thr Ser Gly Lys Ser Tyr Trp Glu Val Asp Val Ser Lys Lys
           355                 360                 365
   Thr Ala Trp Ile Leu Gly Val Cys Ala Gly Phe Gln Pro Asp Ala Met
       370                 375                 380
   Cys Asn Ile Glu Lys Asn Glu Asn Tyr Gln Pro Asn Tyr Gly Tyr Trp
   385                 390                 395                 400
   Val Ile Gly Leu Glu Glu Gly Val Lys Cys Ser Ala Phe Gln Asp Ser
                   405                 410                 415
   Ser Phe His Thr Pro Ser Val Pro Phe Ile Val Pro Leu Ser Val Leu
               420                 425                 430
   Ile Cys Pro Asp Arg Val Gly Val Phe Leu Asp Tyr Glu Ala Cys Thr
           435                 440                 445
   Val Ser Phe Phe Asn Ile Thr Asn His Gly Phe Leu Ile Tyr Lys Phe
       450                 455                 460
   Ser His Cys Ser Phe Ser Gln Pro Val Phe Pro Tyr Leu Asn Pro Arg
   465 470 475 480
   Lys Cys Gly Val Pro Met Thr Leu Cys Ser Pro Ser Ser
   485 490
<210> 29
   <211> 1482
   <212> DNA
   <213> Homo sapiens
<220>
   <221> misc_feature
   <222> (1)..(1482)
   <223> human TRIM5-alpha protein nucleic acid sequence
<400> 29
   atggcttctg gaatcctggt taatgtaaag gaggaggtga cctgccccat ctgcctggaa      60
   ctcctgacac aacccctgag cctggactgc ggccacagct tctgccaagc atgcctcact     120
   gcaaaccaca agaagtccat gctagacaaa ggagagagta gctgccctgt gtgccggatc     180
   agttaccagc ctgagaacat acggcctaat cggcatgtag ccaacatagt ggagaagctc     240
   agggaggtca agttgagccc agaggggcag aaagttgatc attgtgcacg ccatggagag     300
   aaacttctac tcttctgtca ggaggacggg aaggtcattt gctggctttg tgagcggtct     360
   caggagcacc gtggtcacca cacgttcctc acagaggagg ttgcccggga gtaccaagtg     420
   aagctccagg cagctctgga gatgctgagg cagaagcagc aggaagctga agagttagaa     480
   gctgacatca gagaagagaa agcttcctgg aagactcaaa tacagtatga caaaaccaac     540
   gtcttggcag attttgagca actgagagac atcctggact gggaggagag caatgagctg     600
   caaaacctgg agaaggagga ggaagacatt ctgaaaagcc ttacgaactc tgaaactgag     660
   atggtgcagc agacccagtc cctgagagag ctcatctcag atctggagca tcggctgcag     720
   gggtcagtga tggagctgct tcagggtgtg gatggcgtca taaaaaggac ggagaacgtg     780
   accttgaaga agccagaaac ttttccaaaa aatcaaagga gagtgtttcg agctcctgat     840
   ctgaaaggaa tgctagaagt gtttagagag ctgacagatg tccgacgcta ctgggttgat     900
   gtgacagtgg ctccaaacaa catttcatgt gctgtcattt ctgaagataa gagacaagtg     960
   agctctccga aaccacagat aatatatggg gcacgaggga caagatacca gacatttgtg    1020
   aatttcaatt attgtactgg catcctgggc tctcaaagta tcacatcagg gaaacattac    1080
   tgggaggtag acgtgtccaa gaaaactgct tggatcctgg gggtatgtgc tggcttccaa    1140
   cctgatgcaa tgtgtaatat tgaaaaaaat gaaaattatc aacctaaata cggctactgg    1200
   gttatagggt tagaggaagg agttaaatgt agtgctttcc aggatagttc cttccatact    1260
   ccttctgttc ctttcattgt gcccctctct gtgattattt gtcctgatcg tgttggagtt    1320
   ttcctagact atgaggcttg cactgtctca ttcttcaata tcacaaacca tggatttctc    1380
   atctataagt tttctcagtg ttctttttct cagcctgtat ttccatattt aaatcctaga    1440
   aaatgtggag tccccatgac tctgtgctca ccaagctctt ga                       1482
<210> 30
   <211> 30
   <212> DNA
   <213> Artificial Sequence
<220>
   <223> Synthetic: HIV-gag sense primer
<400> 30
   agtgggggga catcaagcag ccatgcaaat 30
<210> 31
   <211> 28
   <212> DNA
   <213> Artificial Sequence
<220>
   <223> Synthetic: HIV-gag antisense primer
<400> 31
   tactagtagt tcctgctatg tcacttcc 28
<210> 32
   <211> 29
   <212> DNA
   <213> Artificial Sequence
<220>
   <223> Synthetic: HIV-gag detection probe
<220>
   <221> misc_feature
   <223> 5' FAM / 3' TAMRA
<400> 32
   atcaatgagg aagctgcaga atgggatag 29
<210> 33
   <211> 25
   <212> DNA
   <213> Artificial Sequence
<220>
   <223> Synthetic: Beta-actin sense primer
<400> 33
   tcacccacac tgtgcccatc tacga 25
<210> 34
   <211> 25
   <212> DNA
   <213> Artificial Sequence
<220>
   <223> Synthetic: Beta-actin anti-sense primer
<400> 34
   cagcggaacc gctcattgcc aatgg 25
<210> 35
   <211> 25
   <212> DNA
   <213> Artificial Sequence
<220>
   <223> Synthetic: Beta-actin detection probe
<220>
   <221> misc_feature
   <223> 5' FAM / 3' TAMRA
<400> 35
   atgccctccc ccatgccatc ctgcg 25
<210> 36
   <211> 19
   <212> DNA
   <213> HIV-1
<220>
   <221> misc_feature
   <222> (1)..(19)
   <223> 350 to 368 5' LTR U3-PromA
<400> 36
   gggactttcc gctggggac 19
<210> 37
   <211> 19
   <212> DNA
   <213> HIV-1
<220>
   <221> misc_feature
   <222> (1)..(19)
   <223> Strain NL4.3, subtypes A, B, D, F, H, J, K (350 to 368 5LTR U3-PromA)
<400> 37
   gggactttcc gctggggac 19
<210> 38
   <211> 18
   <212> DNA
   <213> HIV-1
<220>
   <221> misc_feature
   <222> (1)..(18)
   <223> Subtype C (350 to 368 5LTR U3-PromA)
<400> 38
   gggactttcc actggggc 18
<210> 39
   <211> 19
   <212> DNA
   <213> HIV-1
<220>
   <221> misc_feature
   <222> (1)..(19)
   <223> Subtype G (350 to 368 5LTR U3-PromA)
<400> 39
   gggactttcc gcctgggac 19
<210> 40
   <211> 19
   <212> RNA
   <213> Artificial Sequence
<220>
   <223> Synthetic: RNA duplex sense strand (350 to 368 5 LTR U3-PromA)
<400> 40
   gggacuuucc gcuggggac 19
<210> 41
   <211> 19
   <212> RNA
   <213> Artificial Sequence
<220>
   <223> Synthetic: RNA duplex antisense strand (350 to 368 5 LTR U3-PromA)
<400> 41
   guccccagcg gaaaguccc 19
<210> 42
   <211> 57
   <212> RNA
   <213> Artificial Sequence
<220>
   <223> Synthetic: shRNA (350 to 368 5LTR U3-PromA)
<400> 42
   gggacuuucc gcuggggacc ugugaagcca cagauggggu ccccagcgga aaguccc 57
<210> 43
   <211> 19
   <212> RNA
   <213> Artificial Sequence
<220>
   <223> Synthetic: shRNA Loop sequence
<400> 43
   cugugaagcc acagauggg 19